# EUROPEAN PATENT APPLICATION

(11) **EP 4 800 035 A1**
(43) Date of publication of application: **02.09.2026**
(21) Application number: 24882507.7
(22) Date of filing: 25.10.2024
(51) Int. Cl.: C07K 16/28, A61K 39/395, A61K 47/68, A61P 35/00, C07K 16/46, C12N 15/13, C12N 15/62, C12P 21/08

(54) **ANTIBODY AGAINST INTERLEUKIN-7 RECEPTOR (IL-7R), AND ANTIBODY-DRUG CONJUGATE (ADC) INCLUDING SAID ANTIBODY**

(30) Priority: 26.10.2023 JP 2023184031
(71) Applicant: National Cancer Center, Tokyo 104-0045 (JP)
(72) Inventor: YASUNAGA Masahiro, Kashiwa-shi, Chiba 277-8577 (JP); ANZAI Takahiro, Kashiwa-shi, Chiba 277-8577 (JP)
(74) Representative: Klöckner, Christoph
(86) International application number: PCT/JP2024/038145
(87) International publication number: WO 2025/089399

(57) **Abstract**

The present disclosure relates to an isolated monoclonal antibody or an antigen-binding fragment thereof that binds to human interleukin-7 receptor (IL-7R), a humanized antibody or an antigen-binding fragment thereof, and a pharmaceutical composition comprising the antibody or antigen-binding fragment thereof.

## Description

### TECHNICAL FIELD

The present disclosure relates to antibody against interleukin-7 receptor (IL-7R) and antibody-drug conjugate (ADC) comprising the antibody.

### BACKGROUND ART

IL-7 is known as a cytokine essential for T cell development and homeostasis (Non-Patent Document 1). In addition, IL-7Ralpha slowly undergoes intracellular translocation, and a portion returns to the cell surface while another portion is degraded (Non-Patent Document 1). Stimulation by IL-7 shortens the half-life of IL-7Ralpha from approximately 24 hours to 3 hours (Non-Patent Document 1). In addition, it has been revealed that IL-7R is a cause of steroid resistance in cancer (Non-Patent Document 2). Non-Patent Document 2 suggests that suppressing the downstream signal of IL-7R is effective for the treatment of steroid resistance. Patent Document 1 discloses that ADCs of IL-7R antibodies and cytotoxic agents are effective for the therapy of hematopoietic tumors (for example, leukemia and lymphoma) and inflammatory diseases (for example, rheumatoid arthritis and ulcerative colitis). Patent Document 1 also discloses that cell lines that have acquired steroid resistance highly express IL-7R.

### [PRIOR ART]

### [NON-PATENT DOCUMENTS]

[Non-Patent Document 1] Li et al., 2016, PLOS Medicine, 13(12): e1002200 [Non-Patent Document 2] Henriques et al., 2010, Blood, 115(16): 3269-3277

### [PATENT DOCUMENTS]

[Patent Document 1] WO2018/159808A

### SUMMARY OF THE INVENTION

The present disclosure provides antibody against interleukin-7 receptor (IL-7R) and antibody-drug conjugate (ADC) comprising the antibody. The present disclosure also relates to pharmaceutical compositions comprising the antibody or the ADC.

According to the present disclosure, the following inventions are exemplified.
[1] An isolated monoclonal antibody or an antigen-binding fragment thereof that binds to human interleukin-7 receptor (IL-7R), wherein
   the antibody and the antigen-binding fragment thereof comprise:
   (1) a heavy chain variable region comprising heavy chain CDR1 having the amino acid sequence set forth in SEQ ID NO: 17, heavy chain CDR2 having the amino acid sequence set forth in SEQ ID NO: 18, and heavy chain CDR3 having the amino acid sequence set forth in SEQ ID NO: 19, and a light chain variable region comprising light chain CDR1 having the amino acid sequence set forth in SEQ ID NO: 20, light chain CDR2 having the amino acid sequence set forth in SEQ ID NO: 21, and light chain CDR3 having the amino acid sequence set forth in SEQ ID NO: 22;
   (2) a heavy chain variable region comprising heavy chain CDR1 having the amino acid sequence set forth in SEQ ID NO: 25, heavy chain CDR2 having the amino acid sequence set forth in SEQ ID NO: 26, and heavy chain CDR3 having the amino acid sequence set forth in SEQ ID NO: 27, and a light chain variable region comprising light chain CDR1 having the amino acid sequence set forth in SEQ ID NO: 28, light chain CDR2 having the amino acid sequence set forth in SEQ ID NO: 29, and light chain CDR3 having the amino acid sequence set forth in SEQ ID NO: 30;
   (3) a heavy chain variable region comprising heavy chain CDR1 having the amino acid sequence set forth in SEQ ID NO: 33, heavy chain CDR2 having the amino acid sequence set forth in SEQ ID NO: 34, and heavy chain CDR3 having the amino acid sequence set forth in SEQ ID NO: 35, and a light chain variable region comprising light chain CDR1 having the amino acid sequence set forth in SEQ ID NO: 36, light chain CDR2 having the amino acid sequence set forth in SEQ ID NO: 37, and light chain CDR3 having the amino acid sequence set forth in SEQ ID NO: 38; or
   (4) a heavy chain variable region comprising heavy chain CDR1 having the amino acid sequence set forth in SEQ ID NO: 41, heavy chain CDR2 having the amino acid sequence set forth in SEQ ID NO: 42, and heavy chain CDR3 having the amino acid sequence set forth in SEQ ID NO: 43, and a light chain variable region comprising light chain CDR1 having the amino acid sequence set forth in SEQ ID NO: 44, light chain CDR2 having the amino acid sequence set forth in SEQ ID NO: 45, and light chain CDR3 having the amino acid sequence set forth in SEQ ID NO: 46.
[2] The antibody or the antigen-binding fragment thereof according to the above [1], wherein the antibody or the antigen-binding fragment thereof is a human chimeric antibody or an antigen-binding fragment thereof.
[3] The antibody or the antigen-binding fragment thereof according to the above [1], wherein the antibody or the antigen-binding fragment thereof is a humanized antibody or an antigen-binding fragment thereof.
[4] The antibody or the antigen-binding fragment thereof according to the above [3], wherein the humanized antibody has a heavy chain constant region, an Fc region, and a light chain constant region of a subclass selected from the group consisting of human IgG1, IgG2, and IgG4.
[5] An antibody-drug conjugate comprising
   an antibody or an antigen-binding fragment thereof and a drug, wherein the antibody or fragment is linked to the drug via a linker, and the antibody or fragment comprises the antibody or the antigen-binding fragment thereof according to any one of the above [1] to [4].
[6] A pharmaceutical composition comprising the antibody or the antigen-binding fragment thereof according to any one of the above [1] to [4].
[7] The pharmaceutical composition according to the above [6], wherein the antibody or the antigen-binding fragment thereof has antibody-dependent cellular cytotoxicity activity and/or complement-dependent cytotoxicity activity.
[8] A pharmaceutical composition comprising the antibody-drug conjugate according to the above [5].
[9] The pharmaceutical composition according to any one of the above [6] to [8], for use in treating cancer in a subject in need thereof.
[10] The pharmaceutical composition according to any one of the above [6] to [8], for use in treating an immune disease or a disease resulting from an immune disease in a subject in need thereof.
[21] The antibody or the antigen-binding fragment thereof according to any one of the above [1] to [4], comprising heavy chain framework region 1 (heavy chain FR1), heavy chain FR2, heavy chain FR3, and heavy chain FR4 of an antibody subclass consisting of human IgG1, human IgG2, human IgG3, and human IgG4, and light chain framework region 1 (light chain FR1), light chain FR2, light chain FR3, and light chain FR4 of a human kappa chain or lambda chain.
[22] The antibody or the antigen-binding fragment thereof according to any one of the above [1] to [4], having heavy chain variable region comprising heavy chain framework region 1 (heavy chain FR1), heavy chain FR2, heavy chain FR3, and heavy chain FR4 of one or two or more antibodies selected from antibody group A, and
   a light chain variable region comprising light chain framework region 1 (light chain FR1), light chain FR2, light chain FR3, and light chain FR4 of one or two or more antibodies selected from the above group; or
   a heavy chain variable region and light chain variable region having an amino acid sequence having 90% or more sequence identity with the amino acid sequences of the FR regions of the heavy chain variable region and the light chain variable region.
[23] The antibody or the antigen-binding fragment thereof according to any one of the above [1] to [4], having heavy chain variable region having heavy chain FR1 having the amino acid sequence set forth in SEQ ID NO: 51, heavy chain FR2 having the amino acid sequence set forth in SEQ ID NO: 52, heavy chain FR3 having the amino acid sequence set forth in SEQ ID NO: 53, and heavy chain FR4 having the amino acid sequence set forth in SEQ ID NO: 54, and light chain variable region having light chain FR1 having the amino acid sequence set forth in SEQ ID NO: 55, light chain FR2 having the amino acid sequence set forth in SEQ ID NO: 56, light chain FR3 having the amino acid sequence set forth in SEQ ID NO: 57, and light chain FR4 having the amino acid sequence set forth in SEQ ID NO: 58; or
   heavy chain variable region and light chain variable region having an amino acid sequence having 90% or more sequence identity with the amino acid sequences of the FR regions of the heavy chain variable region and the light chain variable region.
[24] The antibody or the antigen-binding fragment thereof according to any one of the above [1] to [4], having heavy chain variable region having heavy chain FR1 having the amino acid sequence set forth in SEQ ID NO: 59, heavy chain FR2 having the amino acid sequence set forth in SEQ ID NO: 60, heavy chain FR3 having the amino acid sequence set forth in SEQ ID NO: 61, and heavy chain FR4 having the amino acid sequence set forth in SEQ ID NO: 62, and light chain variable region having light chain FR1 having the amino acid sequence set forth in SEQ ID NO: 63, light chain FR2 having the amino acid sequence set forth in SEQ ID NO: 64, light chain FR3 having the amino acid sequence set forth in SEQ ID NO: 65, and light chain FR4 having the amino acid sequence set forth in SEQ ID NO: 66; or
   heavy chain variable region and light chain variable region having an amino acid sequence having 90% or more sequence identity with the amino acid sequences of the FR regions of the heavy chain variable region and the light chain variable region.
[25] An antibody-drug conjugate comprising
   an antibody or an antigen-binding fragment thereof and a drug, wherein the antibody or the fragment is linked to the drug via a linker, and the antibody or the fragment comprises the antibody or the antigen-binding fragment thereof according to any one of the above [21] to [24].
[26] A pharmaceutical composition comprising the antibody or the antigen-binding fragment thereof according to any one of the above [21] to [24].
[27] The pharmaceutical composition according to the above [26], wherein the antibody or the antigen-binding fragment thereof has antibody-dependent cellular cytotoxicity activity and/or complement-dependent cytotoxicity activity.
[28] A pharmaceutical composition comprising the antibody-drug conjugate according to the above [25].
[29] The pharmaceutical composition according to any one of the above [26] to [28], for use in treating cancer in a subject in need thereof.
[30] The pharmaceutical composition according to any one of the above [26] to [28], for use in treating an immune disease or a disease resulting from an immune disease in a subject in need thereof.
[101] A nucleic acid (for example, DNA or messenger RNA (mRNA)) encoding the antibody or the antigen-binding fragment thereof according to any one of the above [1] to [4].
[102] A cell comprising the nucleic acid according to the above [101] (for example, a protein-producing cell such as a Chinese hamster ovary (CHO) cell or a CHO-derived cell, a human embryonic kidney (HEK) cell (for example, a HEK293 cell) or a HEK cell-derived cell).
[103] A nucleic acid comprising the nucleic acid according to the above [101] and a regulatory sequence operably linked to the nucleic acid.
[104] A vector comprising the nucleic acid according to the above [102] or [103].
[105] A cell comprising the vector according to the above [104] (for example, a protein-producing cell such as a Chinese hamster ovary (CHO) cell or a CHO-derived cell, a human embryonic kidney (HEK) cell (for example, a HEK293 cell) or a HEK cell-derived cell).
[201] An isolated multispecific binding peptide (preferably a bispecific binding protein) comprising a binding domain that binds to human interleukin-7 receptor (IL-7R) and a binding domain that binds to a T cell receptor complex, wherein
   the binding domain that binds to IL-7R comprises an antigen-binding fragment of the antibody according to any one of the above [1] to [4] and [21] to [24].
[202] The multispecific binding peptide according to the above [201], wherein the binding domain that binds to a T cell receptor complex comprises an antigen-binding fragment of an antibody that binds to CD3 (for example, a CD3 epsilon chain).
[301] A chimeric antigen receptor (CAR) comprising an antigen-binding fragment of the antibody according to any one of the above [1] to [4] and [21] to [24].
[302] The chimeric antigen receptor according to the above [301], wherein the antigen-binding fragment is an scFv.
[303] The chimeric antigen receptor according to the above [301] or [302], further comprising an extracellular hinge domain, a transmembrane domain, and an activating signal transduction domain.
[304] The chimeric antigen receptor according to the above [303], further comprising a costimulatory molecule signal transduction domain.
[305] The chimeric antigen receptor according to the above [304], wherein the costimulatory molecule signal transduction domain comprises a costimulatory molecule signal transduction domain of one or more costimulatory molecules selected from the group consisting of CD28, 4-1BB, OX40, CD27, and ICOS.
[351] A cell (particularly an immune cell, preferably a T cell or an NK cell) expressing the chimeric antigen receptor according to any one of the above [301] to [305].
[352] A nucleic acid encoding the chimeric antigen receptor according to any one of the above [301] to [305].
[353] A nucleic acid comprising the nucleic acid according to the above [352] and a regulatory sequence operably linked to the nucleic acid.
[354] A vector comprising the nucleic acid according to the above [352] or [353].
[355] A cell comprising the nucleic acid or the vector according to any one of the above [352] to [354].
[371] A composition comprising the cell according to the above [351].
[372] A pharmaceutical composition comprising the cell according to the above [351].
[373] The pharmaceutical composition according to the above [372], for use in treating a subject having cancer, an inflammatory disease, or an immune disease.
[374] The pharmaceutical composition according to the above [372] or [373], for use in treating cancer, an inflammatory disease, or an immune disease in a subject.
[401] The ADC according to any one of the above [5] or [25], wherein the drug is a cytotoxic agent.
[402] The ADC according to any one of the above [5] or [25], wherein the drug is an anticancer agent (for example, a chemotherapeutic agent or a radioisotope).
[403] The ADC according to any one of the above [5] or [25], wherein the drug is a nucleic acid.
[404] The ADC according to any one of the above [5] or [25], wherein the drug is a signal transduction inhibitor (for example, a kinase inhibitor).
[405] The ADC according to any one of the above [5] or [25], wherein the drug is a JAK inhibitor.
[451] The ADC according to any one of the above [5], [25], and [401] to [405], wherein the linker is a cleavable linker or a non-cleavable linker.
[452] The ADC according to the above [451], wherein the linker comprises polyethylene glycol (PEG), valine-citrulline, and p-aminobenzylcarbamate (PABC) via a thiol group of a cysteine residue of the antibody.
[471] A composition comprising the ADC according to any one of the above [401] to [405], [451], and [452].
[472] A pharmaceutical composition comprising the ADC according to any one of the above [401] to [405], [451], and [452].
[473] The pharmaceutical composition according to the above [472], for use in treating a subject having cancer, an inflammatory disease, or an immune disease.
[474] The pharmaceutical composition according to the above [472] or [473], for use in treating cancer, an inflammatory disease, or an immune disease in a subject.

### [BRIEF DESCRIPTION OF THE DRAWINGS]

[FIG. 1] FIG. 1 shows the results of a test confirming the reactivity to IL-7R of various monoclonal antibody clones that bind to IL-7 receptor (IL-7R).
[FIG. 2] FIG. 2 shows the results of a test confirming the reactivity and dose dependency of various monoclonal antibody clones to IL-7R-expressing human cells.
[FIG. 3] FIG. 3 shows the dose dependency of cytotoxicity of antibody-drug conjugates comprising human chimeric antibodies derived from the indicated various antibody clones and a monomethyl auristatin E (MMAE), which is a cytotoxic agent, against cultured cells (specifically, IL-7R-expressing human cells).
[FIG. 4] FIG. 4 shows the antitumor effect of antibody-drug conjugates comprising a human chimeric antibody and monomethyl auristatin E (MMAE), which is a cytotoxic agent, in a xenograft model.
[FIG. 5] FIG. 5 shows the results of a test confirming the reactivity of the indicated human chimeric antibody clones and humanized antibody clones to IL-7R-expressing human cells.
[FIG. 6] FIG. 6 shows the dose dependency of cytotoxicity of antibody-drug conjugates comprising a human chimeric antibody or a humanized antibody and monomethyl auristatin E (MMAE), which is a cytotoxic agent, against cultured cells (specifically, IL-7R-expressing human cells).
[FIG. 7] FIG. 7 shows the antitumor effect of clone 577 chimeric antibody and clone 165 chimeric antibody in the IgG1 format and the IgG4 format against a tumor-bearing model. The vertical axis represents tumor volume, and the horizontal axis represents the days elapsed from the start of treatment.
[FIG. 8] FIG. 8 shows the antitumor effect of various ADCs with PNU-159682 as a payload against cultured tumor cells.
[FIG. 9] FIG. 9 shows the antitumor effect of ADCs with PNU-159682 as a payload against a tumor-bearing model. The vertical axis represents tumor volume, and the horizontal axis represents the days elapsed from the start of treatment.

### DESCRIPTION OF THE EMBODIMENTS

### <Definition of Terms>

As used herein, "subject" means a mammal, and may particularly be a human.

As used herein, "treatment" is used in a meaning including therapy (therapeutic treatment) and prophylaxis (prophylactic treatment). As used herein, "therapy" means treating, curing, preventing, or improving remission of a disease or disorder, or reducing the rate of progression of a disease or disorder. As used herein, "prophylaxis" means reducing the possibility of onset of a disease or condition, or delaying the onset of a disease or condition.

As used herein, "disease" means a condition for which therapy is beneficial. Diseases include, for example, cancer, inflammatory diseases, and immune diseases.

As used herein, "therapeutically effective amount" means an amount of an agent effective for treatment (prophylaxis or therapy) of a disease or condition. A therapeutically effective amount of an agent is capable of reducing the rate of worsening of symptoms of a disease or condition, stopping the worsening of the symptoms, improving the symptoms, curing the symptoms, or suppressing the onset or development of the symptoms. A pharmaceutical composition comprises a therapeutically effective amount of a therapeutically active ingredient. The therapeutically active ingredient may be a pharmaceutically acceptable salt. The therapeutically active ingredient may be a pharmaceutically acceptable solvate (for example, a hydrate). The drug moiety of an ADC may be in a free form or may be a pharmaceutically acceptable salt. The drug moiety of an ADC may also be a pharmaceutically acceptable solvate (for example, a hydrate).

As used herein, "resistance" means showing no efficacy to a therapy (for example, showing no significant efficacy). As used herein, "sensitivity" means showing efficacy to a therapy. Reducing resistance has the same meaning as increasing sensitivity, and increasing resistance has the same meaning as reducing sensitivity. As used herein, for example, steroid resistance may be defined as a failure to achieve complete remission even with daily administration of prednisolone for 4 weeks or more in the case of nephrotic syndrome. Also herein, with regard to other inflammatory/autoimmune diseases or malignant diseases such as leukemia and malignant lymphoma, steroid resistance may be treated as a failure to improve symptoms or worsening of symptoms even with administration of various therapeutic steroids such as prednisolone, methylprednisolone, dexamethasone, and betamethasone. A physician can understand and grasp what resistance is based on knowledge in the field of steroid therapy, and can appropriately determine whether a subject has resistance or not.

As used herein, "IL-7R" means interleukin-7 receptor. When IL-7 (interleukin-7) binds to IL-7R, JAK1, JAK2, and JAK3 are activated, and autophosphorylation of the receptor occurs. STAT binds to the phosphorylated receptor, is phosphorylated by JAK, and dimerizes. It is believed that transcriptional activation of genes occurs by the dimerized STAT. IL-7R has high internalization activity of the molecule itself, and internalizes from the membrane, is degraded or recycled and re-expressed on the membrane. Because antibodies against IL-7R are internalized into cells together with IL-7R, antibodies targeting IL-7R are suitable for delivering drugs into cells. In addition, the appearance of IL-7R-positive monocytes/macrophages in inflammatory/autoimmune diseases and the like has been reported. In this case, it is considered that inflammatory/autoimmune diseases can be effectively controlled by delivering drugs that inhibit JAK1/JAK2/JAK3/TYK2, which are highly expressed in monocytes/macrophages, to monocytes/macrophages (References 1-3).

As used herein, "blocking" means neutralizing the binding of two proteins. Blocking includes partial dissociation in addition to complete dissociation. For example, blocking can also be said to occur when the binding of two proteins is reduced by 50% or more.

As used herein, "steroid" means a steroidal anti-inflammatory drug (SAIDs). In steroids, mainly glucocorticoids or derivatives thereof serve as the active ingredient. When steroids bind to glucocorticoid receptor (GRalpha) in cells, GRalpha dissociates from hsp90, forms a dimer, translocates into the nucleus, and performs transcriptional regulation. Examples of synthetic steroids include cortisols such as hydrocortisone and hydrocortisone succinate; prednisolones such as prednisolone, methylprednisolone, and methylprednisolone succinate; triamcinolones such as triamcinolone and triamcinolone acetonide; dexamethasone; and betamethasone. All of these are derivatives of glucocorticoid and function intracellularly by a mechanism similar to that of glucocorticoid.

As used herein, "antibody" means an immunoglobulin and includes polyclonal antibodies and monoclonal antibodies. As the antibody, a monoclonal antibody is preferred.

The origin of the antibody is not particularly limited, and examples include non-human animal antibodies (for example, non-human mammalian antibodies) and human antibodies. In addition, the antibody may be a chimeric antibody or a humanized antibody. In addition, the antibody may be a bispecific antibody. As the origin of the antibody, a humanized antibody or a human antibody is preferred.

Antibodies have subclasses of IgG, IgA, IgE, IgM, and IgD. In addition, human IgG has subclasses of IgG1, IgG2, IgG3, and IgG4. In pharmaceuticals, human IgG1, IgG2, and IgG4 are preferably used, and in human chimeric antibodies and humanized antibodies, antibodies having sequences of these subclasses may be used.

As the antibody, a chimeric monoclonal antibody, a humanized monoclonal antibody, or a human monoclonal antibody is preferred. When used as a pharmaceutical, a chimeric monoclonal antibody is preferably used, and a humanized monoclonal antibody or a human monoclonal antibody is more preferably used, and each is preferably isolated. Note that chimeric monoclonal antibodies and humanized monoclonal antibodies can be prepared from non-human animal antibodies by a method known per se.

A humanized antibody has a heavy chain variable region and a light chain variable region comprising CDRs of a non-human animal antibody and framework regions of a human antibody, a heavy chain constant region and an Fc region of a human antibody, and a light chain constant region. A humanized antibody is typically prepared by replacing the CDRs of a human antibody with the corresponding CDRs of a non-human animal antibody. A humanized antibody may also be prepared by introducing amino acid modifications selected from the group consisting of addition, insertion, deletion, and substitution into one or more of the CDRs or framework regions. Amino acid modifications are typically performed to enhance or restore the affinity of a humanized antibody for an antigen.

A human chimeric antibody has a heavy chain variable region and a light chain variable region of a non-human animal antibody, and a heavy chain constant region and an Fc region, as well as a light chain constant region of a human antibody. It may be prepared by replacing the heavy chain constant region and Fc region, as well as the light chain constant region of a human antibody with the corresponding regions of a non-human animal.

An antibody has a structure in which two heavy chains and two light chains are associated. A heavy chain consists of a heavy chain variable region (VH), a heavy chain constant region (CH1, CH2, and CH3), and a hinge region located between the heavy chain variable region and the heavy chain constant region. A light chain consists of a light chain variable region (VL) and a light chain constant region (CL). The heavy chain variable region and the light chain variable region each have three complementarity-determining regions (CDRs), by which the antigen specificity of the antibody is characterized. The CDRs are referred to as heavy chain CDR1, heavy chain CDR2, and heavy chain CDR3, and light chain CDR1, light chain CDR2, and light chain CDR3, from the N-terminal side of the heavy chain and the light chain, respectively. The antibody includes a full-length antibody.

As used herein, "bispecific binding protein" is a protein having two or more domains that bind to two different antigens or epitopes and capable of binding to the two different antigens. The domains are typically derived from antibodies and preferably have a heavy chain variable region and a light chain variable region of an antibody. A bispecific binding protein has a first binding domain that binds to a first antigen and a second binding domain that binds to a second antigen, and the first antigen and the second antigen are different, or bind to different epitopes of the same molecule. The first binding domain and the second binding domain are each derived from an antibody and have, for example, a heavy chain variable region and a light chain variable region of an antibody that binds to the first antigen and the second antigen, respectively. As the bispecific binding protein, for example, an antibody or an antigen-binding fragment thereof having, in addition to the heavy chain variable region and the light chain variable region of an antibody that binds to the first antigen and the second antigen, preferably a heavy chain constant region and an Fc region, and a light chain constant region of a subclass selected from the group consisting of human IgG1, IgG2, and IgG4 can be mentioned. In addition, as the bispecific binding protein, a fusion protein of an scFv that binds to a first antigen and an scFv that binds to a second antigen can be mentioned.

As used herein, "antigen-binding fragment" means a portion of an antibody in which binding to an antigen is maintained. An antigen-binding fragment may comprise the heavy chain variable region or the light chain variable region, or both, of the antibody of the present disclosure. An antigen-binding fragment may be chimerized or humanized. Examples of the antigen-binding fragment include Fab, Fab', F(ab')₂, Fv, scFv (single-chain Fv), diabody, sc(Fv)₂ (single-chain (Fv)₂), and half-molecule Ig. The present disclosure may use these antigen-binding fragments of antibodies. Such antibody fragments can be obtained, without limitation, for example, by treating an antibody with an enzyme. For example, digesting an antibody with papain can yield Fab. Alternatively, digesting an antibody with pepsin can yield F(ab')_{2,} and further reducing this can yield Fab'. These can be expressed and purified as recombinant proteins in prokaryotic cell and eukaryotic cell expression systems.

As used herein, "antibody-drug conjugate" (ADC) means a substance in which a monoclonal antibody or an antigen-binding fragment thereof (hereinafter, sometimes simply referred to as "antibody or the like") and a drug are directly or indirectly linked. In ADCs, the monoclonal antibody or the like and the drug can be linked via an appropriate linker. ADCs bind to membrane components on the cell membrane (for example, transmembrane proteins such as receptors), are taken up into cells by endocytosis or internalization, and can be separated from the antibody or the like and released into cells. By introducing a cleavable linker between the antibody or the like and the drug intracellularly, it is possible to cleave the linker intracellularly, for example, in endosomes, to dissociate the drug from the antibody or the like and release it into the cytoplasm. In addition, even if the ADC does not internalize into cells, it is possible to produce the expected pharmacological effect of the drug (particularly small molecules such as cytotoxic agents) against target cells through the bystander effect. When a cytotoxic agent is used as the drug, it is possible to kill the cells to which the drug has been delivered. As the cytotoxic agent, chemotherapeutic agents, radioisotopes, and toxins can be used. In addition, when a signal transduction inhibitor is used as the drug, the signal transduction can be inhibited within the cells to which the drug has been delivered. Examples of the signal transduction inhibitor include kinase inhibitors (see, for example, WO2020/130125A). Examples of the signal transduction inhibitor include inhibitors of the downstream signal pathway of IL-7R, and particularly JAK inhibitors. By using a JAK inhibitor as a payload of an ADC targeting IL-7R, the JAK inhibitor can be selectively applied to IL-7R-expressing cells. Therefore, it can be expected that this can reduce the side effects of the JAK inhibitor on IL-7R non-expressing cells and/or enhance the effect on IL-7R-expressing cells.

<Antibody and Antigen-Binding Fragment Thereof of the Present Disclosure> According to the present disclosure, an isolated monoclonal antibody or an antigen-binding fragment thereof that binds to human interleukin-7 receptor (IL-7R) is provided. In a preferred embodiment, the antibody and the antigen-binding fragment thereof comprise:
(1) a heavy chain variable region comprising heavy chain CDR1 having the amino acid sequence set forth in SEQ ID NO: 17, heavy chain CDR2 having the amino acid sequence set forth in SEQ ID NO: 18, and heavy chain CDR3 having the amino acid sequence set forth in SEQ ID NO: 19, and a light chain variable region comprising light chain CDR1 having the amino acid sequence set forth in SEQ ID NO: 20, light chain CDR2 having the amino acid sequence set forth in SEQ ID NO: 21, and light chain CDR3 having the amino acid sequence set forth in SEQ ID NO: 22;
(2) a heavy chain variable region comprising heavy chain CDR1 having the amino acid sequence set forth in SEQ ID NO: 25, heavy chain CDR2 having the amino acid sequence set forth in SEQ ID NO: 26, and heavy chain CDR3 having the amino acid sequence set forth in SEQ ID NO: 27, and a light chain variable region comprising light chain CDR1 having the amino acid sequence set forth in SEQ ID NO: 28, light chain CDR2 having the amino acid sequence set forth in SEQ ID NO: 29, and light chain CDR3 having the amino acid sequence set forth in SEQ ID NO: 30;
(3) a heavy chain variable region comprising heavy chain CDR1 having the amino acid sequence set forth in SEQ ID NO: 33, heavy chain CDR2 having the amino acid sequence set forth in SEQ ID NO: 34, and heavy chain CDR3 having the amino acid sequence set forth in SEQ ID NO: 35, and a light chain variable region comprising light chain CDR1 having the amino acid sequence set forth in SEQ ID NO: 36, light chain CDR2 having the amino acid sequence set forth in SEQ ID NO: 37, and light chain CDR3 having the amino acid sequence set forth in SEQ ID NO: 38; or
(4) a heavy chain variable region comprising heavy chain CDR1 having the amino acid sequence set forth in SEQ ID NO: 41, heavy chain CDR2 having the amino acid sequence set forth in SEQ ID NO: 42, and heavy chain CDR3 having the amino acid sequence set forth in SEQ ID NO: 43, and a light chain variable region comprising light chain CDR1 having the amino acid sequence set forth in SEQ ID NO: 44, light chain CDR2 having the amino acid sequence set forth in SEQ ID NO: 45, and light chain CDR3 having the amino acid sequence set forth in SEQ ID NO: 46.

In a preferred embodiment, the antibody and the antigen-binding fragment thereof may be a human chimeric antibody, or an antigen-binding fragment thereof. In a preferred embodiment, the antibody and the antigen-binding fragment thereof may be a humanized antibody and an antigen-binding fragment thereof. As active ingredients of a pharmaceutical composition, human chimeric antibodies and antigen-binding fragments thereof are preferably used, and humanized antibodies and antigen-binding fragments thereof may be more preferably used.

Antibodies derived from non-human mammals can be humanized using techniques well known in the art (for example, techniques described in Winter et al., Immunol. Today, 14:43-46 (1993)). Humanized monoclonal antibodies can be engineered by using recombinant DNA technology that replaces the CH1, CH2, CH3, hinge domain, and/or framework domain of an antibody derived from a non-human mammal with corresponding human sequences (for example, techniques described in WO92/02190, and U.S. Patent Nos. 5,530,101, 5,585,089, 5,693,761, 5,693,792, 5,714,350, and 5,777,085).

In a preferred embodiment, a human chimeric antibody has a heavy chain constant region and an Fc region derived from human IgG1, and a human light chain constant region. In a preferred embodiment, a human chimeric antibody has a heavy chain constant region and an Fc region derived from human IgG2, and a human light chain constant region. In a preferred embodiment, a human chimeric antibody has a heavy chain constant region and an Fc region derived from human IgG4, and a human light chain constant region.

In a preferred embodiment, a humanized antibody has a heavy chain constant region, an Fc region, and a light chain constant region of a subclass selected from the group consisting of human IgG1, IgG2, IgG3, and IgG4. In a preferred embodiment, a humanized antibody has a heavy chain constant region, an Fc region, and a light chain constant region of a subclass selected from the group consisting of human IgG1, IgG2, and IgG4. In a preferred embodiment, a humanized antibody has a heavy chain constant region, an Fc region, and a light chain constant region of human IgG1. In a preferred embodiment, a humanized antibody has a heavy chain constant region, an Fc region, and a light chain constant region of human IgG2. In a preferred embodiment, a humanized antibody has a heavy chain constant region, an Fc region, and a light chain constant region of human IgG4.

In an embodiment, a humanized antibody or a chimeric antibody may have antibody-dependent cellular cytotoxicity (ADCC) activity or complement-dependent cytotoxicity (CDC) activity. For this purpose, a humanized antibody or a chimeric antibody may have an Fc region derived from human IgG1. ADCC activity and CDC activity can be measured by a conventional method.

The heavy chain variable region of an antibody typically comprises heavy chain framework region 1 (heavy chain FR1), heavy chain CDR1, heavy chain FR2, heavy chain CDR2, heavy chain FR3, heavy chain CDR3, and heavy chain FR4, and this is also the case for the heavy chain variable region of a humanized antibody. In addition, the light chain variable region of an antibody typically comprises light chain framework region 1 (light chain FR1), light chain CDR1, light chain FR2, light chain CDR2, light chain FR3, light chain CDR3, and light chain FR4, and this is also the case for the light chain variable region of a humanized antibody.

In a preferred embodiment, heavy chain framework region 1 (heavy chain FR1), heavy chain CDR1, heavy chain FR2, heavy chain CDR2, heavy chain FR3, heavy chain CDR3, and heavy chain FR4, and light chain framework region 1 (light chain FR1), light chain CDR1, light chain FR2, light chain CDR2, light chain FR3, light chain CDR3, and light chain FR4 may be heavy chain framework region 1 (heavy chain FR1), heavy chain CDR1, heavy chain FR2, heavy chain CDR2, heavy chain FR3, heavy chain CDR3, and heavy chain FR4, and light chain framework region 1 (light chain FR1), light chain CDR1, light chain FR2, light chain CDR2, light chain FR3, light chain CDR3, and light chain FR4 of one or two or more antibodies selected from the group (antibody group A) consisting of abituzumab, abrilumab, adalimumab, alemtuzumab, alirocumab, anifrolumab, atezolizumab, bapineuzumab, belimumab, benralizumab, bevacizumab, bimagrumab, blosozumab, bococizumab, briakinumab, brodalumab, canakinumab, carlumab, certolizumab, cixutumumab, clazakizumab, codrituzumab, crenezumab, dacetuzumab, daclizumab, dalotuzumab, daratumumab, denosumab, drozitumab, duligotuzumab, dupilumab, eculizumab, efalizumab, elderlumab, elotuzumab, emibetuzumab, enokizumab, epratuzumab, etrolizumab, evolocumab, farletuzumab, fezakinumab, fezakinumab, ficlatuzumab, figitumumab, fletikumab, foralumab, fresolimumab, fulranumab, ganitumab, gantenerumab, gemtuzumab, gevokizumab, glembatumumab, golimumab, guselkumab, ibalizumab, imgatuzumab, inotuzumab, ipilimumab, ixekizumab, lampalizumab, lebrikizumab, lenzilumab, linzilumab, lirilumab, matuzumab, mavrilimumab, mepolizumab, mogamulizumab, motavizumab, muromonab, natalizumab, necitumumab, nimotuzumab, nivolumab, obinutuzumab, ocrelizumab, octatumumab, olaratumab, olokizumab, omalizumab, onartuzumab, otelixizumab, otraltzumab, ozanezumab, palivizumab, panitumumab, panobacumab, parsatuzumab, patritumab, pembrolizumab, pertuzumab, pinatuzumab, polatuzumab, ponezumab, radretumab, ramucirumab, ranibizumab, reslizumab, rilotumumab, robatumumab, romosozumab, sarilumab, secukinumab, seribantumab, sifalimumab, simtuzumab, simtuzumab, tabalumab, tanezumab, teplizumab, tigatuzumab, tildrakizumab, tocilizumab, tovetumab, tralokinumab, trastuzumab, tremelimumab, urelumab, ustekinumab, vedolizumab, veltuzumab, visilizumab, zalutumumab, and zanolimumab. Heavy chain framework regions 1 to 4 may each be derived from different antibodies or from the same antibody. Light chain framework regions 1 to 4 may each be derived from different antibodies or from the same antibody. Heavy chain framework regions 1 to 4 and light chain framework regions 1 to 4 may each be derived from different antibodies or from the same antibody.

In a preferred embodiment, heavy chain FR1, heavy chain FR2, heavy chain FR3, and heavy chain FR4 are derived from a heavy chain constant region of a subclass selected from the group consisting of human IgG1, IgG2, IgG3, and IgG4. In a preferred embodiment, heavy chain FR1, heavy chain FR2, heavy chain FR3, and heavy chain FR4 are derived from a heavy chain constant region of a subclass selected from the group consisting of human IgG1, IgG2, and IgG4. In a preferred embodiment, light chain FR1, light chain FR2, light chain FR3, and light chain FR4 are derived from a kappa chain or a lambda chain.

In a preferred embodiment, heavy chain FR1, heavy chain FR2, heavy chain FR3, and heavy chain FR4 have the amino acid sequence set forth in SEQ ID NO: 7, the amino acid sequence set forth in SEQ ID NO: 8, the amino acid sequence set forth in SEQ ID NO: 9, and the amino acid sequence set forth in SEQ ID NO: 10, respectively. In a preferred embodiment, light chain FR1, light chain FR2, light chain FR3, and light chain FR4 have the amino acid sequence set forth in SEQ ID NO: 11, the amino acid sequence set forth in SEQ ID NO: 12, the amino acid sequence set forth in SEQ ID NO: 13, and the amino acid sequence set forth in SEQ ID NO: 14, respectively.

In a preferred embodiment, heavy chain FR1, heavy chain FR2, heavy chain FR3, and heavy chain FR4 have the amino acid sequence set forth in SEQ ID NO: 51, the amino acid sequence set forth in SEQ ID NO: 52, the amino acid sequence set forth in SEQ ID NO: 53, and the amino acid sequence set forth in SEQ ID NO: 54, respectively. In a preferred embodiment, light chain FR1, light chain FR2, light chain FR3, and light chain FR4 have the amino acid sequence set forth in SEQ ID NO: 55, the amino acid sequence set forth in SEQ ID NO: 56, the amino acid sequence set forth in SEQ ID NO: 57, and the amino acid sequence set forth in SEQ ID NO: 58, respectively.

In a preferred embodiment, heavy chain FR1, heavy chain FR2, heavy chain FR3, and heavy chain FR4 have the amino acid sequence set forth in SEQ ID NO: 59, the amino acid sequence set forth in SEQ ID NO: 60, the amino acid sequence set forth in SEQ ID NO: 61, and the amino acid sequence set forth in SEQ ID NO: 62, respectively. In a preferred embodiment, light chain FR1, light chain FR2, light chain FR3, and light chain FR4 have the amino acid sequence set forth in SEQ ID NO: 63, the amino acid sequence set forth in SEQ ID NO: 64, the amino acid sequence set forth in SEQ ID NO: 65, and the amino acid sequence set forth in SEQ ID NO: 66, respectively.

In a preferred embodiment, the antibody and the antigen-binding fragment thereof have:
(1) a heavy chain variable region comprising heavy chain CDR1 having the amino acid sequence set forth in SEQ ID NO: 17, heavy chain CDR2 having the amino acid sequence set forth in SEQ ID NO: 18, and heavy chain CDR3 having the amino acid sequence set forth in SEQ ID NO: 19, and a light chain variable region comprising light chain CDR1 having the amino acid sequence set forth in SEQ ID NO: 20, light chain CDR2 having the amino acid sequence set forth in SEQ ID NO: 21, and light chain CDR3 having the amino acid sequence set forth in SEQ ID NO: 22,
   the heavy chain variable region has heavy chain FR1, heavy chain FR2, heavy chain FR3, and heavy chain FR4, and heavy chain FR1, heavy chain FR2, heavy chain FR3, and heavy chain FR4 have the amino acid sequence set forth in SEQ ID NO: 51, the amino acid sequence set forth in SEQ ID NO: 52, the amino acid sequence set forth in SEQ ID NO: 53, and the amino acid sequence set forth in SEQ ID NO: 54, respectively,
   the light chain variable region has light chain FR1, light chain FR2, light chain FR3, and light chain FR4, and light chain FR1, light chain FR2, light chain FR3, and light chain FR4 have the amino acid sequence set forth in SEQ ID NO: 55, the amino acid sequence set forth in SEQ ID NO: 56, the amino acid sequence set forth in SEQ ID NO: 57, and the amino acid sequence set forth in SEQ ID NO: 58, respectively.

In a preferred embodiment, the antibody and the antigen-binding fragment thereof have: (4) a heavy chain variable region comprising heavy chain CDR1 having the amino acid sequence set forth in SEQ ID NO: 41, heavy chain CDR2 having the amino acid sequence set forth in SEQ ID NO: 42, and heavy chain CDR3 having the amino acid sequence set forth in SEQ ID NO: 43, and a light chain variable region comprising light chain CDR1 having the amino acid sequence set forth in SEQ ID NO: 44, light chain CDR2 having the amino acid sequence set forth in SEQ ID NO: 45, and light chain CDR3 having the amino acid sequence set forth in SEQ ID NO: 46,
the heavy chain variable region has heavy chain FR1, heavy chain FR2, heavy chain FR3, and heavy chain FR4, and heavy chain FR1, heavy chain FR2, heavy chain FR3, and heavy chain FR4 have the amino acid sequence set forth in SEQ ID NO: 59, the amino acid sequence set forth in SEQ ID NO: 60, the amino acid sequence set forth in SEQ ID NO: 61, and the amino acid sequence set forth in SEQ ID NO: 62, respectively,
the light chain variable region has light chain FR1, light chain FR2, light chain FR3, and light chain FR4, and light chain FR1, light chain FR2, light chain FR3, and light chain FR4 have the amino acid sequence set forth in SEQ ID NO: 63, the amino acid sequence set forth in SEQ ID NO: 64, the amino acid sequence set forth in SEQ ID NO: 65, and the amino acid sequence set forth in SEQ ID NO: 66, respectively.

Heavy chain FR1 to FR4 and light chain FR1 to FR4 may each have an amino acid sequence having 90% or more, 91% or more, 92% or more, 93% or more, 94% or more, 95% or more, 96% or more, 97% or more, 98% or more, or 99% or more sequence identity with the amino acid sequences of the above corresponding heavy chain FR1 to FR4 and light chain FR1 to FR4. Alternatively, heavy chain FR1 to FR4 and light chain FR1 to FR4 may each have 1 to several (for example, 1 to 5, 1 to 4, 1 to 3, or 1 to 2) amino acid mutations relative to the amino acid sequences of the above corresponding heavy chain FR1 to FR4 and light chain FR1 to FR4. The amino acid mutations may be one or more selected from the group consisting of amino acid addition, insertion, substitution, and deletion.

In an embodiment, an antigen-binding fragment of any of the above humanized antibodies is provided. In a preferred embodiment, the antigen-binding fragment may comprise, for example, Fab, Fab', or scFv. In a preferred embodiment, the antigen-binding fragment comprises scFv. In a preferred embodiment, the antigen-binding fragment may be, for example, Fab, Fab', or scFv, and may be, for example, scFv. These fragments may also be fused with other proteins or other fragments. For example, by fusing with other fragments that bind to other epitopes or antigens, a multispecific binding protein (for example, a bispecific binding protein) can be obtained. In addition, these fragments (typically scFv) or multispecific binding proteins (for example, bispecific binding proteins) comprising the fragments may be fused with other proteins and incorporated into chimeric antigen receptors (first generation, second generation, and third generation).

As used herein, "chimeric antigen receptor" (CAR) is a chimeric molecule having an antigen-binding fragment of an antibody (particularly, scFv) and an activation domain of an immune cell. A CAR is generally a molecule in which an scFv, an extracellular hinge domain, a transmembrane domain (for example, CD8alpha or CD28), and an activating signal transduction domain (for example, CD3zeta) are linked. A CAR can be introduced into a cell and expressed on the cell surface. Cells expressing a CAR can be targeted to a specific antigen. CARs are introduced into immune cells such as T cells or NK cells, and target immune cells such as T cells or NK cells to target cells such as cancer cells. In first generation CARs, an scFv, an extracellular hinge domain, a transmembrane domain (for example, CD8alpha or CD28), and an activating signal transduction domain (for example, CD3zeta) were linked, but second generation CARs further comprise a costimulatory molecule signal transduction domain for activation of immune cells into which the CAR has been introduced. As the costimulatory molecule signal transduction domain, costimulatory factors such as CD28, 4-1BB, OX40, CD27, and ICOS have been used. In third generation CARs, multiple costimulatory factors (for example, combinations of the above costimulatory factors) are incorporated. In this way, improvements have been made to CARs for sustained proliferation of immune cells into which CARs have been introduced in vivo. It is preferred that all domains other than the scFv portion are derived from human proteins.

### <Antibody-Drug Conjugate (ADC) of the Present Disclosure>

In an embodiment, an antibody or an antigen-binding fragment thereof may be in the form of a conjugate with a drug. According to the present disclosure, a conjugate (ADC) comprising an antibody or an antigen-binding fragment thereof and a drug is provided, and the antibody may be an antibody or an antigen-binding fragment thereof of the present disclosure. The drug may be a molecule desired to be delivered to IL-7R-positive cells by the antibody. The drug is not particularly limited, and includes biologically active substances, and preferably includes small molecules, cytokines, and nucleic acids. The drug also includes contrast agents. Examples of small molecules include cytotoxic agents and signal transduction inhibitors (preferably inhibitors of the downstream signal pathway of IL-7R, preferably JAK inhibitors). Examples of cytotoxic agents include, but are not limited to, toxins, anticancer agents, and radioisotopes.

Examples of toxins include Pseudomonas exotoxin (PE) or a cytotoxic fragment thereof (for example, PE38), diphtheria toxin, ricin A, and the like.

Examples of anticancer agents include chemotherapeutic agents (for example, anticancer agents such as commercially available anticancer agents, for example, auristatins (auristatin E, auristatin F phenylenediamine (AFP), monomethyl auristatin E, monomethyl auristatin F, and derivatives thereof), maytansinoids DM1 and DM4 and derivatives thereof), camptothecins (SN-38, topotecan, and exatecan and derivatives thereof (for example, deruxtecan)), DNA minor groove binding agents (enediynes, lexitropsins, duocarmycins, and derivatives thereof), taxanes (paclitaxel and docetaxel and derivatives thereof), polyketides (discodermolide and derivatives thereof), anthraquinone-based (mitoxantrone and derivatives thereof), benzodiazepines (pyrrolobenzodiazepines, indolinobenzodiazepines, and oxazolidinobenzodiazepines and derivatives thereof), vinca alkaloids (vincristine, vinblastine, vindesine, and vinorelbine and derivatives thereof), doxorubicins (doxorubicin, morpholino-doxorubicin, PNU-159682 (CAS Registry Number: 202350-68-3), and cyanomorpholino-doxorubicin and derivatives thereof), cardiac glycosides (digitoxin and derivatives thereof), calicheamicin, epothilone, cryptophycin, cemadotin, cemadotin, rhizoxin, netropsin, combretastatin, eleutherobin, etoposide, T67 (tularik), and nocodazole), radioisotopes (for example, ³²P, ⁶⁰C, 9⁰Y, ¹¹¹In, ¹³¹I, ¹²⁵I, ¹⁵³Sm, ¹⁸⁶Re, ¹⁸⁸Re, and ²¹²Bi), and toxins (for example, diphtheria toxin A, Pseudomonas endotoxin, ricin, saporin, and the like), and these can be used as cytotoxic agents in the ADCs of the present disclosure. Any cytotoxic agent used for the treatment of cancer can be used.

The radioisotope is not particularly limited, and includes, for example, therapeutic nuclides. The radioisotope is not particularly limited, and includes, for example, ³²P, ¹⁴C, ⁶⁰C, ¹¹¹I, ¹²⁵I, ³H, ¹³¹I, ²¹¹At, ⁹⁰Y, ¹⁸⁶Re, ¹⁸⁸Re, ²¹²Bi, and the like.

The JAK inhibitor is not particularly limited as long as it is a molecule capable of inhibiting JAK, and includes ruxolitinib, tofacitinib, baricitinib, pacritinib, peficitinib, upadacitinib, and filgotinib. The JAK inhibitor is preferably capable of inhibiting one or more selected from the group consisting of JAK1, JAK2, JAK3, and TYK2.

Examples of cytokines include proteins such as cytokines that activate immunocompetent cells (for example, human interleukin-2, human granulocyte macrophage colony-stimulating factor, human macrophage colony-stimulating factor, and human interleukin-12).

Examples of nucleic acids include natural nucleic acids such as natural DNA and natural RNA, antisense oligonucleotide (ASO), siRNA, shRNA, microRNA, gapmer, mixmer, modified nucleic acid such as modified DNA and modified RNA, artificial nucleic acid, and combinations thereof. Examples of modified nucleic acids include fluorescent dye-modified nucleic acids, biotinylated nucleic acids, and nucleic acids into which a cholesteryl group has been introduced. RNA may be 2'-O-methyl modified or 2'-fluoro modified or 2'-methoxyethyl (MOE) modified on the base to enhance stability, and the phosphodiester bond of the nucleic acid backbone may be replaced with a phosphorothioate bond. Examples of artificial nucleic acids include nucleic acids in which the oxygen atom at the 2' position and the carbon atom at the 4' position are bridged. Examples of such artificial nucleic acids include locked nucleic acid (LNA), which is a bridged DNA in which the oxygen atom at the 2' position and the carbon atom at the 4' position are bridged via a methylene, ENA in which the oxygen atom at the 2' position and the carbon atom at the 4' position are bridged via an ethylene, BNA^{COC} in which the oxygen atom at the 2' position and the carbon atom at the 4' position are bridged via -CH₂OCH₂-, bridged nucleic acids (BNA) such as BNA^{NC} in which the oxygen atom at the 2' position and the carbon atom at the 4' position are bridged via -NR-CH₂- {wherein R is methyl or a hydrogen atom}, cMOE in which the oxygen atom at the 2' position and the carbon atom at the 4' position are bridged via -CH₂(OCH₃)-, cEt in which the oxygen atom at the 2' position and the carbon atom at the 4' position are bridged via -CH₂(CH₃)-, AmNA in which the carbon atoms at the 2' position and the 4' position are bridged via an amide, scpBNA in which the oxygen atom at the 2' position and the carbon atom at the 4' position are bridged via a methylene and a cyclopropane is formed at the 6' position, and peptide nucleic acids (PNA) in which N-(2-aminoethyl)glycine polymerized via amide bonds forms the backbone instead of deoxyribose or ribose. Examples of RNA include artificial RNAs for gene silencing such as siRNA and shRNA, non-coding RNAs such as microRNA (miRNA) and aptamers, and natural RNAs such as mRNA. These RNAs may be modified to be stabilized in vivo.

The target of the drug in the conjugate of the present disclosure is not particularly limited, and includes, for example, the following targets.
Clusterin gene, Nucleolin gene, AKT1 protein kinase gene, BIRC5 gene, MAGEC1 gene, MAGEC2 gene, CTAG1 gene, TPBG gene, Hsp27 gene, beta-Catenin gene, CXCL12 (SDF-1) gene, STAT-3 gene, PKN3 gene, PLK1 gene, mutant KRAS (G12D) gene, Grb-2 gene, Androgen receptor gene, TGFbeta gene (TGFbeta-1 gene, TGFbeta-2 gene, TGFbeta-3 gene), STAT-3 gene, VEGF gene, KSP (Eg5) gene, CEBPA gene, Nek2 gene, p53 gene, MUC1 gene, TPBG gene, HIF-1alpha gene, RPN2 gene, EphA2 gene, RRM1 gene, CDC45 gene, six-1 gene, IGF-1 receptor gene, HoxA1 gene, IGFBP-2 gene, IGFBP-5 gene, EGF receptor gene, Raf-1 gene, mTOR gene, Bcl-2 gene, Casein kinase-2 gene, KRAS gene, c-Myc gene, COX-2 gene, beta-3 tubin gene, ITCH gene, VEGF gene, VEGF receptor 2 gene, SIP1 gene, AGT gene, ERV-9 LTR gene, EVI1 gene, TNF-alpha gene, PAX-2 gene, Srpx2 gene, IRS-1 gene, Survivin gene, DUSP6 gene, HPV E6/E7 gene, HSPA9 gene, mitochondrial RNA (non-coding mitochondrial RNA), EWS FLI1 gene, SRC-3 gene, MDR1 gene, NTRK1 gene, NTRK2 gene, MDX3 gene, NR2F6 gene, MYD88 gene, NOTCH1 gene, beta-3 integrin gene, c-FLIP gene, MADD gene, HER2 gene, CCAT2 gene, CTCFL gene, HIF-2alpha gene, BMI-1 gene, NETO-2 gene, CTFR gene, PD-1 gene, PD-L1 gene, PD-L2 (B7-DC (CD273)) gene, CLTA4 gene, HLA gene (HLA-A, HLA-B, HLA-C, HLA-DR, HLA-DP, HLA-DQ, HLA-E, HLA-G), MCH gene, gene, 4-1BB gene, 4-1 BBL gene, CD3 gene (CD3alpha, CD3beta, CD3gamma, CD3delta, CD3epsilon, CD3zeta gene, IL-6 gene, IL-17 gene, IL-23 gene, ICOS gene, CD70 gene, CD27 gene, OX40 gene, OX40L gene, TL1A gene, DR3 gene, GITRL gene, GITR gene, CD30L gene, CD30 gene, TIM1 gene, TIM1L gene, TIM4 gene, SLAM gene, CD48 gene, CD58 gene, CD2 gene, CD155 gene, CD112 gene, CD226 gene, CD80 (B7-1) gene, CD86 (B7-2) gene, B7-H2 gene, LIGHT gene, HVEM gene, CD40 gene, CD40L gene, Galectin 9 gene, CD113, Collagen gene, CD160 gene, LAG3 gene, PSA gene, PSMA gene, PSCA gene, STEAP gene, BIRC5 gene, MAGEC1 gene, MAGEC2 gene, CTAG1 gene, TPBG gene, or molecules such as proteins encoded by these genes, or mRNAs encoding proteins encoded by these genes, proteins known as the HDGF family (prototype protein, HDGF; HRP-1, HRP-2, HRP-3, HRP-4 (HRP = HDGF Related Protein); and LEDGF (particularly, HRP-3)), TrkB receptor, or genes or mRNAs encoding these receptors, miRNAs such as miR-34, miR-34a, miR-16, miR-155, miR-17, miR-17-92, miR-215, let-7, miR-34, miR-10b, miR-3157, miR-34, miR-7, miR-21, miR-574-5p, miR-221, miR-484, miR-205, miR-210, miR-3189-3p, miR-3151, miR-199, miR-101, miR-96, and miR-182.

In the ADC of the present disclosure, the drug and the antibody are linked by a linker. The linker may be a cleavable linker or a non-cleavable linker. Examples of cleavable linkers include linkers having in their structure an -S-S- bond that cleaves under the intracellular reducing environment (for example, SS linkers, DMSS linkers), linkers having in their structure a hydrazone bond that cleaves at low pH in endosomes, linkers having in their structure an orthoester bond, and linkers having in their structure a peptide bond that is cleaved by cathepsin B (for example, linkers having a valine-citrulline bond within the molecule (Val-Cit linkers)), and these may be preferably used in the present disclosure. Examples of cleavable linkers also include, in addition to the above, linkers having in their structure a sugar chain that is cleaved by a glycolytic enzyme such as glucuronidase, and these may be preferably used in the present disclosure.

Examples of non-cleavable linkers include linkers that do not have in their structure a cleavable bond site under intracellular environments (for example, under the low pH environment in endosomes and under the intracellular reducing environment) (sometimes referred to herein as maleimide linkers), and these may be preferably used in the present disclosure.

As an example of a cleavable linker, a linker having a succinimidyl group obtained by reacting the sulfur atom of a cysteine residue of an antibody with a maleimide group, a first spacer, valine-citrulline, and a second spacer can be mentioned. The first spacer may be a polyalkylene glycol (preferably polyethylene glycol). In addition, the second spacer may be p-aminobenzylcarbamate (PABC). As an example of a cleavable linker, a linker comprising a succinimidyl group obtained by reacting the sulfur atom of a cysteine residue of an antibody with a maleimide group, a polyalkylene glycol (preferably polyethylene glycol), valine-citrulline, and p-aminobenzylcarbamate (PABC) can be mentioned. In an embodiment, the drug-to-antibody ratio (DAR) may be a natural number of 1 to 8. The DAR indicates how many drugs are linked to one antibody.

In an embodiment, an ADC may have the following structure. 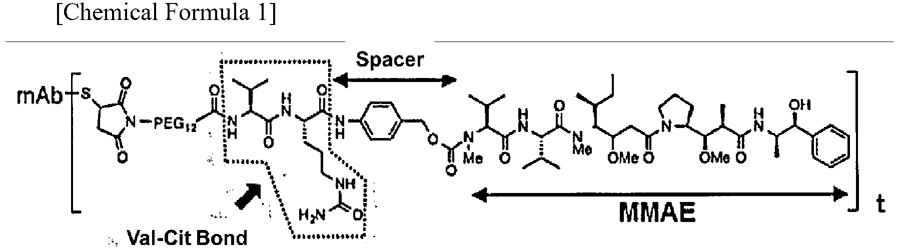 {In the formula, "mAb" represents a monoclonal antibody, "PEG₁₂" represents a polyethylene glycol with a degree of polymerization of 12, and "t" represents the drug-to-antibody ratio. Here, t is a natural number of 1 to 8 when the mAb is IgG1. An alkyl having 1 to 10 carbon atoms ( for example, a lower alkyl having 1 to 6 carbon atoms) may be used instead of PEG.}

With regard to the above ADC, a person skilled in the art can understand that an antigen-binding fragment thereof can be used in place of the antibody. That is, an ADC may comprise an antigen-binding fragment of an antibody and a drug, and the fragment, and the drug may be linked via the above linker. The drug may be directly linked to the linker, or the drug may be encapsulated in a drug delivery carrier (for example, a lipid nanoparticle), and the lipid nanoparticle may be directly linked to the linker. The linker may be directly linked to a thiol group of a cysteine residue or an amino group of a lysine residue (preferably a thiol group). In a preferred embodiment, the linker is linked via a succinimidyl group to a thiol group of a cysteine residue of an antibody.

According to the present disclosure, an antibody-drug conjugate (ADC) of an antibody that binds to IL-7R and PNU-159682 is also provided. The antibody and PNU-159682 can be linked via a linker. The antibody can be any antibody as long as it is capable of binding to IL-7R expressed on the cell membrane. Examples of the antibody include the antibodies disclosed herein, and antibodies having the heavy chain CDR1 to CDR3 and light chain CDR1 to CDR3 of anti-IL-7R antibodies such as GSK2618960 and PF-06342674, and these may be incorporated into ADCs. Even if 1 to several (for example, 1 to 5, 1 to 3, or 1) amino acid mutations exist in the amino acid sequence, the binding affinity for IL-7R is not necessarily lost, and antibodies having 1 to several (for example, 1 to 5, 1 to 3, or 1) amino acid mutations in the amino acid sequence can be preferably used in the present disclosure as long as they have binding affinity for IL-7R.

As an example, the structure of a conjugate of an antibody and PNU-159682 is shown below. In this conjugate, PEG4, a valine-citrulline dipeptide, p-aminobenzyl, and a crosslink formed by a crosslinker are linked to the sulfur atom of a cysteine residue of an antibody via a succinimidyl group, and a payload PNU-159682 is conjugated thereto. When the antibody is IgG1, the drug-to-antibody ratio is 1 to 8. When the antibody is IgG4, the drug-to-antibody ratio may be 1 to 8. In addition, between the two carbamate bonds formed by the crosslinker, the moiety may be a substituted or unsubstituted alkylene, alkynylene, or alkenylene (for example, may be methyl, ethyl, or propyl), and one or more carbons may be replaced by a heteroatom, for example, a heteroatom selected from the group consisting of S, N, and O.

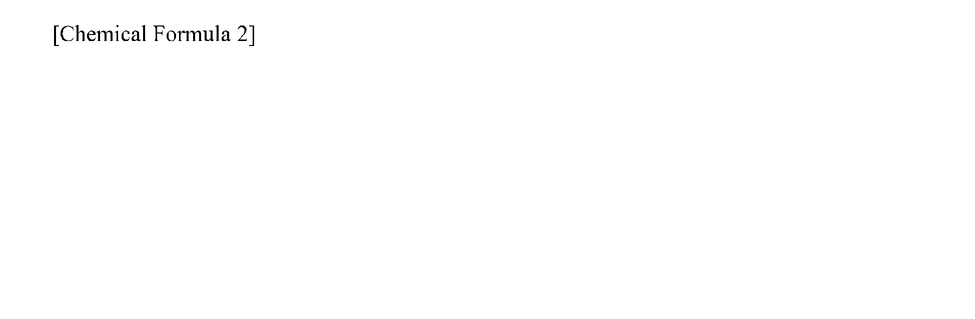

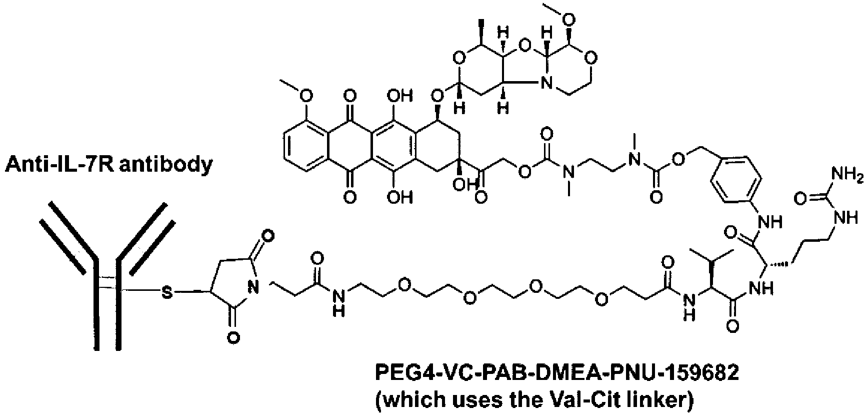

### <Bispecific Binding Protein of the Present Disclosure>

According to the present disclosure, an antibody or an antigen-binding fragment thereof of the present disclosure may be a bispecific protein. That is, an antibody or an antigen-binding fragment thereof of the present disclosure has a first binding domain that binds to a first antigen or epitope and a second binding domain that binds to a second antigen or epitope. In an embodiment, the first antigen and the second antigen are different. In an embodiment, the first antigen and the second antigen are the same, but the epitope of the first binding domain and the epitope of the second binding domain are different. In a preferred embodiment where the first antigen and the second antigen are the same, the first binding domain and the second binding domain can simultaneously bind to the antigen (do not compete for binding to the antigen).

In a preferred embodiment, the first antigen binds to IL-7R, and the second antigen binds to a T cell antigen (for example, a T cell receptor complex, preferably CD3, for example, a CD3 epsilon chain (CD3epsilon)). In this embodiment, the bispecific antibody links a tumor cell or an immune cell with a T cell, and thereby can damage the tumor cell or the immune cell.

In a preferred embodiment, the first binding domain and the second binding domain can simultaneously bind to IL-7R. In this embodiment, the bispecific binding protein may have ADCC activity. In this embodiment also, the bispecific binding protein may or may not have ADCC activity, but may be in the form of a conjugate with a drug (ADC). For details of the ADC, see the section on Antibody-Drug Conjugate (ADC) of the Present Disclosure above.

The first binding domain and the second binding domain may each comprise a heavy chain variable region and a light chain variable region of an antibody. The first binding domain and the second binding domain may each comprise a Fab fragment or an scFv fragment of an antibody.

In a preferred embodiment, the bispecific binding protein may be a whole IgG-type bispecific antibody. In a whole IgG-type bispecific antibody, the first binding domain and the second binding domain each comprise a Fab fragment. In a preferred embodiment, in the bispecific binding protein, the first binding domain and the second binding domain comprise scFv fragments, and preferably the first binding domain and the second binding domain are linked via a peptide linker (for example, a GS linker and a Whitlow linker (see, for example, US2019/0092818A)).

### <Pharmaceutical Composition of the Present Disclosure>

According to the present disclosure, a pharmaceutical composition comprising an antibody or an antigen-binding fragment thereof of the present disclosure is provided. According to the present disclosure, a pharmaceutical composition comprising an ADC of the present disclosure is also provided. According to the present disclosure, a pharmaceutical composition comprising a bispecific binding protein of the present disclosure is further provided. The pharmaceutical composition may further comprise a pharmaceutically acceptable excipient.

Examples of the pharmaceutically acceptable excipient include, without limitation, salts, solvents (for example, water and ethanol), buffers, sugars and sugar alcohols, surfactants, isotonizing agents, preservatives, antioxidants, chelating agents, and excipients. The pharmaceutical composition of the present disclosure may be formulated as a pharmaceutical for intravenous, subcutaneous, intraperitoneal, or intratumoral administration in accordance with a conventional method. According to the present disclosure, the above conjugate may be prepared as a pharmaceutical using a vehicle suitable for administration (for example, sterile pyrogen-free water) before use.

The pharmaceutical composition of the present disclosure can be used, for example, for the treatment of tumors, inflammatory diseases, or immune diseases.

In many cancer cells, IL-7R is positive. Therefore, many cancers can be targets for therapy by the pharmaceutical of the present disclosure. In addition, in order to identify a highly effective target population, in an embodiment of the present disclosure, the cancer may be an IL-7R-positive cancer or a cancer confirmed to be IL-7R-positive. Examples of IL-7R-positive cancers include leukemia, lymphoma, lung cancer, pancreatic cancer, head and neck cancer, prostate cancer, bladder cancer, breast cancer, esophageal cancer, gastric cancer, colorectal cancer, uterine cancer, ovarian cancer, skin cancer, thyroid cancer, thymic cancer, renal cancer, testicular cancer, penile cancer, liver cancer, biliary tract cancer, brain tumors, bone and soft tissue tumors, retroperitoneal tumors, angiosarcoma and lymphangiosarcoma, and metastatic cancers thereof (for example, metastatic solid tumors). In many cancers, for example, leukemia, lymphoma, advanced cancers, and metastatic cancers, IL-7R is positive. Whether IL-7R is positive or negative can be readily determined using techniques such as immunostaining and FACS using an anti-IL-7R antibody.

The pharmaceutical composition of the present disclosure may be used for treating IL-7R-positive cells (for example, cancer cells, immune cells, inflammatory cells) that are resistant to therapy that blocks the binding of IL-7R and IL-7.

Furthermore, WO2018/159808A discloses that IL-7R is involved in the acquisition of steroid resistance. In particular, IL-7R becomes positive in steroid-resistant cells. Therefore, according to the present disclosure, a pharmaceutical composition for use in treating a steroid-resistant disease or condition, comprising an antibody-drug conjugate comprising an antibody or an antigen-binding fragment thereof of the present disclosure and a drug, is provided. In an embodiment, a pharmaceutical composition for use in reducing steroid resistance of a disease or condition, comprising an antibody-drug conjugate comprising an antibody or an antigen-binding fragment thereof of the present disclosure and a drug, is provided. In an embodiment, the steroid-resistant disease or condition is cancer, an inflammatory disease, or an immune disease. Furthermore, WO2018/159808A discloses that IL-7R expression is enhanced after steroid treatment. Particularly after steroid treatment, IL-7R-positive cells tend to survive, and therefore the efficacy of the conjugate of the present disclosure is enhanced. Even if IL-7R-negative cells survive (for example, in a patient after steroid treatment), a therapeutic effect can be expected. This is because a bystander effect of the PDC can be expected to kill IL-7R-negative cells in the vicinity of IL-7R-positive cells.

In an embodiment, the cells to be treated may be steroid-resistant IL-7R-positive cells. Therefore, the pharmaceutical composition of the present disclosure may be used for treating a subject having steroid-resistant IL-7R-positive cells (for example, cancer cells, inflammatory cells, immune cells).

In an embodiment, a pharmaceutical composition for use in treating a disease or condition such as cancer, an inflammatory disease, or an immune disease in a subject who has undergone steroid therapy, comprising the antibody-drug conjugate of the present disclosure, is provided.

In an embodiment, it is also possible to administer steroids while reducing steroid resistance with the pharmaceutical composition of the present disclosure. Therefore, in an embodiment of the present disclosure, a pharmaceutical composition for use in treating a steroid-resistant disease or condition, comprising an antibody-drug conjugate of an antibody or an antigen-binding fragment thereof of the present disclosure and a cytotoxic agent, for use in combination with a steroid, is provided. Alternatively, in the present disclosure, a pharmaceutical composition for use in reducing steroid resistance of a disease or condition (or increasing steroid sensitivity of a disease or condition), comprising an antibody-drug conjugate of an antibody or an antigen-binding fragment thereof of the present disclosure and a cytotoxic agent, is provided. In these embodiments, the subject may be a subject having a steroid-resistant disease or condition.

Furthermore, WO2018/159808A discloses that an antibody-drug conjugate of an antibody or an antigen-binding fragment thereof of the disclosure and a cytotoxic agent can suppress lymph node enlargement in a subject with cancer. Therefore, according to the present disclosure, a pharmaceutical composition for use in suppressing lymph node enlargement in a subject with cancer, comprising an antibody-drug conjugate comprising an antibody or an antigen-binding fragment thereof of the present disclosure and a drug, is provided.

According to WO2018/159808A, it was also shown that antibody-drug conjugates comprising an antibody of the disclosure and a drug have a symptom-reducing effect in inflammatory diseases such as ulcerative colitis and rheumatoid arthritis. This suggests that IL-7R-positive cells are strongly involved in the onset or maintenance of inflammatory diseases, and means that killing IL-7R-positive cells in an inflammatory state is useful for alleviating inflammation. Therefore, according to the present specification, a pharmaceutical composition for use in treating an inflammatory disease, comprising an antibody-drug conjugate comprising an antibody or an antigen-binding fragment thereof of the present disclosure and a drug, is provided. As used herein, inflammatory diseases include collagen diseases, and also include autoimmune diseases, type I diabetes, multiple sclerosis, systemic lupus erythematosus, allergic diseases, rejection after organ transplantation, and graft-versus-host disease. Inflammatory diseases further include inflammatory bowel diseases such as Crohn's disease. In addition, other autoimmune diseases and collagen diseases include autoimmune diseases or collagen diseases selected from Sjogren's syndrome, scleroderma, dermatomyositis, polyarteritis nodosa, mixed connective tissue disease, Cogan's syndrome, polymyalgia rheumatica, adult-onset Still's disease, giant cell arteritis, antiphospholipid antibody syndrome, Guillain-Barre syndrome, myasthenia gravis, autoimmune hepatitis, autoimmune pancreatitis, primary biliary cholangitis, Takayasu arteritis, Goodpasture syndrome, rapidly progressive glomerulonephritis, autoimmune hemolytic anemia, autoimmune neutropenia, idiopathic thrombocytopenic purpura, Graves' disease, Hashimoto's disease, primary hypothyroidism, idiopathic Addison's disease, pemphigus, pustular psoriasis, psoriasis vulgaris, bullous pemphigoid, herpes gestationis, linear IgA bullous dermatosis, epidermolysis bullosa acquisita, alopecia areata, vitiligo vulgaris, Vogt-Koyanagi-Harada disease, autoimmune optic neuropathy, autoimmune inner ear disease, idiopathic azoospermia, and recurrent pregnancy loss. Type IV allergies involving lymphocytes include rejection reactions in organ transplantation, and bronchial asthma caused by innate lymphoid cells. In many inflammations, lymphocytes are IL-7R-positive. Specifically, since they are positive in acquired immune system lymphocytes, namely T lymphocytes and B lymphocytes, as well as in innate immune system lymphocytes, many inflammatory diseases caused by these cells may be targets for therapy by the pharmaceutical composition of the present disclosure.

In the present disclosure, the inflammatory disease may be a disease or condition that is resistant to steroids or resistant to therapy that blocks the binding of IL-7R and IL-7.

In an aspect of the present disclosure, a method of treating cancer, an inflammatory disease, or an immune disease in a subject in need thereof is provided, comprising administering to the subject an antibody-drug conjugate comprising an antibody or an antigen-binding fragment thereof of the present disclosure and a drug. The conjugate may be administered in a therapeutically effective amount. In an embodiment of the present disclosure, the subject may be a subject resistant to steroid therapy or a subject who has undergone steroid therapy.
In an embodiment of the present disclosure, a method of treating cancer, an inflammatory disease, or an immune disease in a subject in need thereof is provided, comprising administering to the subject an antibody-drug conjugate comprising an antibody or an antigen-binding fragment thereof of the present disclosure and a drug, and administering to the subject a steroidal anti-inflammatory drug. In an embodiment of the present disclosure, the subject may be a subject resistant to steroid therapy or a subject who has undergone steroid therapy.

In an embodiment of the present disclosure, a method of treating a steroid-resistant disease or condition in a subject in need thereof is provided, comprising administering to the subject an antibody-drug conjugate comprising an antibody or an antigen-binding fragment thereof of the present disclosure and a drug.

In an embodiment of the present disclosure, a method of reducing steroid resistance of a disease or condition in a subject in need thereof is provided, comprising administering to the subject an antibody-drug conjugate comprising an antibody or an antigen-binding fragment thereof of the present disclosure and a drug.

In an aspect of the present disclosure, the use of an antibody or an antigen-binding fragment thereof of the present disclosure in the manufacture of a medicament for use in treating cancer, an inflammatory disease, or an immune disease, comprising an antibody-drug conjugate comprising the antibody or the antigen-binding fragment thereof of the present disclosure and a drug, is provided. In an aspect of the present disclosure, the use of an antibody or an antigen-binding fragment thereof of the present disclosure and a cytotoxic agent in the manufacture of a medicament for use in treating cancer, an inflammatory disease, or an immune disease, comprising an antibody-drug conjugate comprising the antibody or the antigen-binding fragment thereof of the present disclosure and a drug, is provided. In an aspect of the present disclosure, the use of an antibody-drug conjugate comprising an antibody or an antigen-binding fragment thereof of the present disclosure and a drug in the manufacture of a medicament for use in treating cancer, an inflammatory disease, or an immune disease is provided.

In an aspect of the present disclosure, the use of an antibody-drug conjugate comprising an antibody or an antigen-binding fragment thereof of the present disclosure and a drug in a method for treating cancer, an inflammatory disease, or an immune disease is provided.

In the present disclosure, instead of killing IL-7R-expressing cells by ADC, the expression of IL-7R in IL-7R-expressing cells may be suppressed. Therefore, in an aspect of the present disclosure, a pharmaceutical composition comprising an expression inhibitor of IL-7R for use in treating cancer, an inflammatory disease, or an immune disease is provided. In the present disclosure, a pharmaceutical composition comprising an expression inhibitor of IL-7R for use in treating a steroid-resistant disease or condition is also provided. In the present disclosure, a pharmaceutical composition comprising an expression inhibitor of IL-7R for use in reducing steroid resistance of a disease or condition is further provided. In the present disclosure, a pharmaceutical composition for use in treating a steroid-resistant disease or condition, comprising an expression inhibitor of IL-7R, for use in combination with a steroid, is further provided. In the present disclosure, a pharmaceutical composition comprising an expression inhibitor of IL-7R for use in suppressing lymph node enlargement in a subject with cancer is further provided.

Examples of expression inhibitors of IL-7R include siRNA, shRNA, and antisense oligos against IL-7R, and these can be used in the present disclosure. Examples of expression inhibitors of IL-7R also include expression vectors comprising DNA encoding siRNA or shRNA.

Also according to the present disclosure, instead of an ADC, a bispecific binding protein that binds to IL-7R-expressing cells and a T cell receptor (TCR) complex (preferably CD3, for example, CD3 epsilon chain (CD3epsilon)) may be used. For details of such a bispecific binding protein, see the section on Bispecific Binding Protein of the Present Disclosure. According to the present disclosure, a pharmaceutical composition comprising a bispecific binding protein of the present disclosure is provided. The excipient to be added to the pharmaceutical composition and the uses of the pharmaceutical composition are as described above.

Also according to the present disclosure, instead of an ADC, T cells expressing a chimeric antigen receptor that binds to IL-7R (CAR-T cells) or NK cells (CAR-NK cells) may be used. When a CAR binds to cells expressing a target antigen, it can activate CAR-expressing cells. According to the present disclosure, a pharmaceutical composition comprising CAR-expressing cells (for example, T cells (for example, cytotoxic T cells) or NK cells) of the present disclosure is provided. The excipient to be added to the pharmaceutical composition and the uses of the pharmaceutical composition are as described above.

### EXAMPLES

### Immunization of IL-7R KO Mice

Recombinant human IL-7R (R&D Systems) with an Fc Tag added at the C-terminus, produced in HEK293 cells, was administered as an immunogen to IL-7R KO mice provided by Professor Ikuta of the Division of Immunoregulation, Institute for Frontier Life and Medical Sciences, Kyoto University. The antigen was diluted using sterile Phosphate Buffered Saline (PBS) to prepare a 40 µg/ml antigen solution and placed in a 1 ml syringe. An equal volume of Freund's Complete Adjuvant (Difco) was placed in another 1 ml syringe, and the respective syringes were connected with an adapter to prepare an emulsion. For the first immunization, 100 µl of the obtained emulsion was administered intraperitoneally. For the second and subsequent immunizations, the immunogen was adjusted to 250 µg/ml with sterile PBS, and an equal volume of GERBU ADJUVANT 100 (Nacalai Tesque) was mixed in a 1.5 ml tube and administered using a 1 ml syringe. The immunogen was administered intraperitoneally at 100µl.

Blood was collected from the tail vein of the immunized mice. The blood was centrifuged at 13,000 x g, 10 minutes, 4 degrees C, and the supernatant was collected and used as a sample. Antibody titer measurement was performed by indirect ELISA using samples serially diluted 2-fold from 500-fold dilution to 64,000-fold dilution. Recombinant Human IL-7 Ralpha Fc Chimera (R&D Systems) was diluted using 0.1 M Phosphate Buffer and 50 µl/well of a solution adjusted to a final concentration of 0.1 µg/ml was added to 96-well immunoplates (MaxiSorp(TM), Thermo Fisher Scientific). The plates were then left standing at room temperature for 1 hour to immobilize. After washing 3 times with 200 µl/well of TBS-T, PBS/0.1% Tween20/0.25% skim milk (BD) was added at 200 µl/well as a blocking solution, and the plates were left standing at room temperature for 30 minutes. The solution was discarded, and after 3 washes with TBS-T, serially diluted samples using the blocking solution were added at 50 µl/well, and the plates were left standing at room temperature for 1 hour. The samples were discarded, and after 3 washes, secondary antibody diluted 5,000-fold in the blocking solution was added at 50 µl/well and left standing at room temperature for 30 minutes. Peroxidase-conjugated AffiniPure Goat antimouse IgG (H+L) (Jackson ImmunoResearch) was used as the secondary antibody. The solution was discarded, washed 3 times with TBS-T, and 100 µl/ml of the chromogenic substrate solution (0.2 M sodium citrate/TMB/30% H2O2) was added, and the reaction was carried out at room temperature for 15 minutes. The reaction was stopped by adding 30 µl/well of 2N H2SO4. Absorbance at a wavelength of 450 nm was then measured with a Spectra Max paradigm (Molecular Devices).

Cell Fusion and Preparation of Anti-IL-7R Antibody-Producing Hybridomas Mouse myeloma cell line P3X63-Ag8.653 (hereinafter referred to as "x63") was purchased from the JCRB Cell Bank. The cells were cultured in a medium prepared by adding 50 ml of heat-inactivated Fetal Bovine Serum (FBS, Gibco) and 10 ml of 100 units/ml Penicillin-100 µg/ml Streptomycin-250 ng/ml Amphotericin B (Wako) to 440 ml of RPMI 1640 (Wako) in a 37 degrees C, 5% CO2 incubator (Sanyo). When the cells reached a semi-confluent state occupying approximately 80% of a 150 mm dish (Corning), passage was performed in a clean bench. The cells were detached by pipetting and placed in a 15 ml centrifuge tube (Corning). The cells were centrifuged using a centrifuge (Universal Centrifuge 5800, Kubota) at 270 x g, 3 min, 4 degrees C, the supernatant was removed, and the cells were suspended in 5 ml of RPMI 1640. A new 150 mm dish was prepared, and an appropriate amount of cell suspension was added thereto, and the dish was gently shaken. This dish was cultured in a CO2 incubator.

The mouse was subjected to cardiac blood collection using a 1 mL syringe (Terumo) under inhalation anesthesia and was exsanguinated. Thereafter, the spleen was surgically excised from the mouse and immersed in a medium prepared by adding 200 units/ml Penicillin-200 µg/ml Streptomycin-500 ng/ml Amphotericin B to RPMI 1640. For infection prevention, the spleen was immersed in 70% ethanol for a few seconds, washed with RPMI 1640, and then transferred to a 100 mm dish containing RPMI 1640. RPMI 1640 was injected into the spleen using a 10 mL syringe (Terumo) and a 22G needle (Terumo) to extract splenocytes. The extract was then passed through an EASY strainer 70 µmmesh (Greiner). The collected cell suspension was centrifuged at 270 x g, 5 min, room temperature, the supernatant was removed, and the cells were suspended in 10 ml of RPMI 1640. This washing with RPMI 1640 was repeated twice, and the cells were suspended in 5 ml of RPMI 1640. Next, P3X63-Ag8.653 to be fused with splenocytes was prepared. The cells were detached by pipetting, centrifuged at 270 x g, 3 min, room temperature, the supernatant was removed, and the cells were suspended in 10 ml of RPMI 1640. This washing with RPMI 1640 was repeated twice, and the cells were suspended in 5 ml of RPMI 1640. The cells were mixed at a ratio of lymph node cells:P3X63-Ag8.653 = 5:1 to 7:1, centrifuged at 270 x g, 3 min, room temperature, and the supernatant was removed. 1.8 g of PEG 4000 was dissolved in 2 ml of RPMI 1640, and 1 ml of PEG 4000 solution filtered through a MILLEX-GV Syringe Driven Filter Unit 0.22 µm was added at a rate of 1 ml/1 minute to the cells, which had been well loosened by tapping, while stirring with a vortex. Next, RPMI 1640 was added at a rate of 1 ml/1 minute for 2 ml, then 4 ml/1 minute for 8 ml, while stirring with a vortex. The cells were centrifuged at 170 x g, 5 min, room temperature, and the supernatant was removed. A Fusion medium was prepared with the composition described in Table 2-2 (see Table 1), and the cells were suspended in the Fusion medium and seeded in Costar 96-Well Cell Culture Plates (Corning).

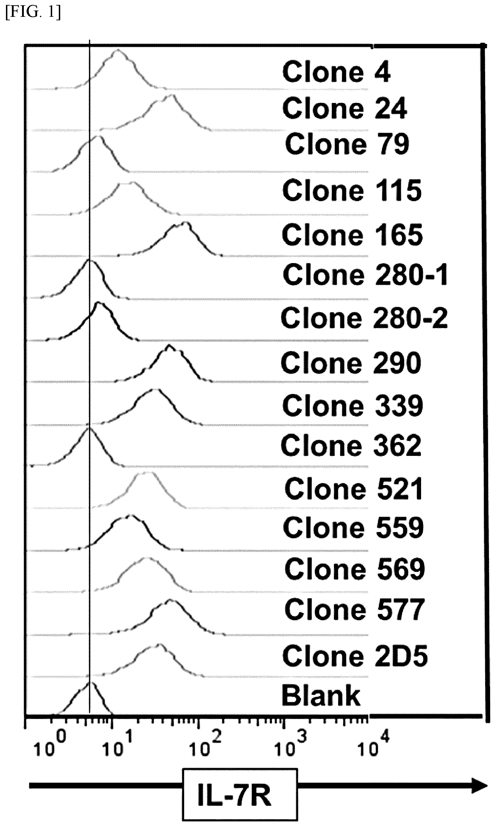

One week after cell fusion, screening was performed by Flow Cytometry and ELISA using culture supernatants. Limiting dilution was performed in wells that were IL-7R-positive and had strong binding to the IL-7R-positive cell line NALM6, each clone was isolated, and binding to the IL-7R-positive cell line NALM6 was again measured by Flow Cytometry (see FIG. 1).

**[Table 1]**

| Table **1** Composition of fusion medium | |
|---|---|
| Reagents | Volime |
| FBS | 100ml |
| RPMI 1640 | 500ml |
| 100 units/ml Penicillin-100 µg/ml | 11.6 ml |
| Streptomycin-250 ng/ml Amphotericin B | |
| HT Supplement(100×) | 9.5ml |
| 45w/v% D-glucose | 6ml |
| mouse IL-6 | 2ml |
| HAT Supplement | 3ml |
| S-clone | 4ml |
| total | 1149ml |

### Cloning of Antibody Genes

Total RNA was extracted from hybridoma cell clones using RNeasy mini Kit (QIAGEN). Heavy chain variable region and light chain variable region gene fragments of the antibody were obtained by the 5'-RACE method using SMARTer RACE cDNAAmplification Kit (Clontech). KOD One (TOYOBO) was used as a polymerase in the first step, and Platinum Taq (Thermo Fisher) was used in the second step for amplification.

### Primer Sequences Used

Forward primer (SEQ ID NO: 1): CTAATACGACTCACTATAGGGCAAGCAGTGGTATCAACGCAGAGT
Reverse primer 1 (SEQ ID NO: 2): GGACTCTGGGATCATTTACCCGGAGAGT
Reverse primer 2 (SEQ ID NO: 3): CCTTAGGAGGGAAGATTGGAAGGAGCT

Gene fragments were cloned into the pTA2 vector (TOYOBO), and the sequences were determined by a DNA sequencer. The following shows the amino acid sequences of the heavy chain variable region and the light chain variable region of each decoded clone. In addition, the frames in each sequence indicate the complementarity-determining regions (CDRs) estimated by Kabat numbering (see Kabat et al., Ann. NY Acad Sci 190:382-93 (1971) and "Sequence of Proteins of Immunological Interest", US Dept. Health and Human Services, 1983).

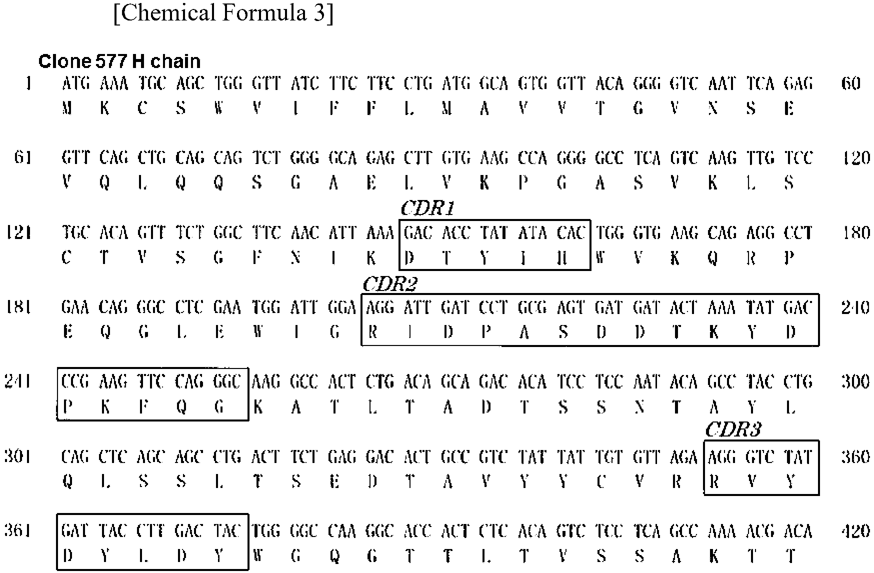

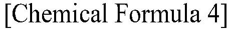

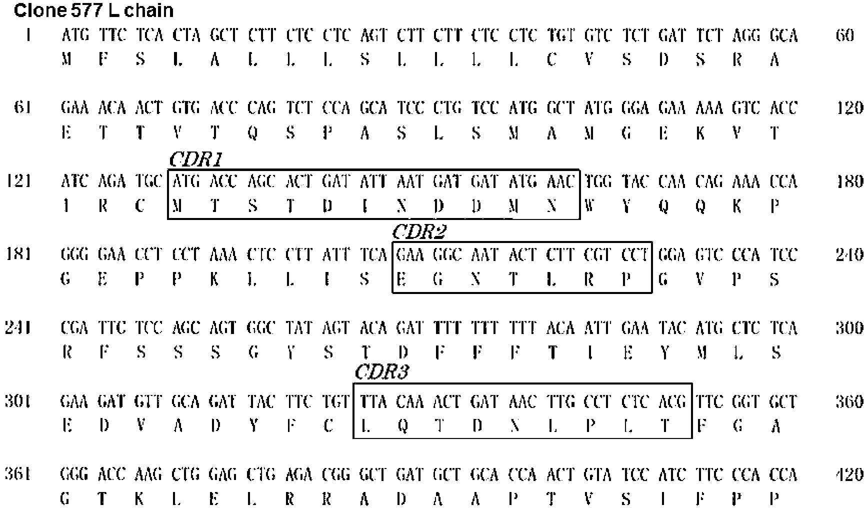

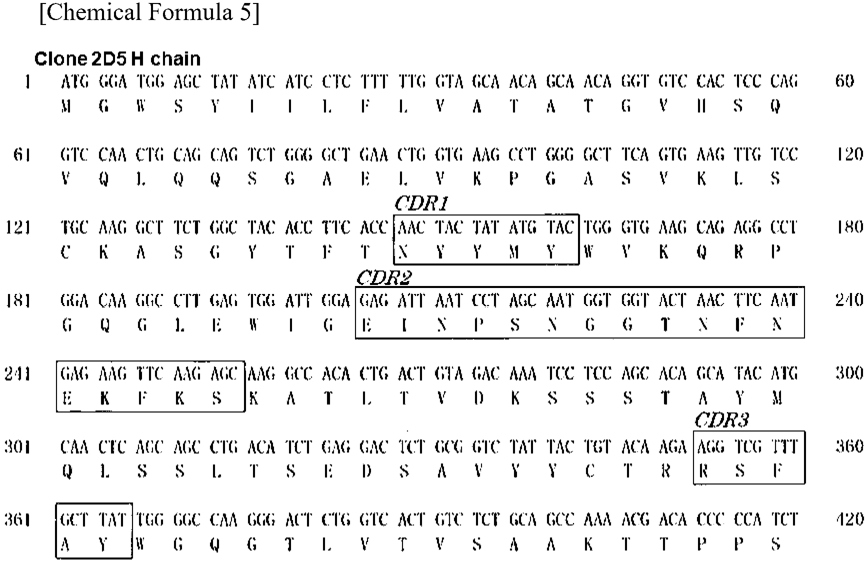

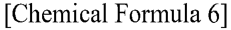

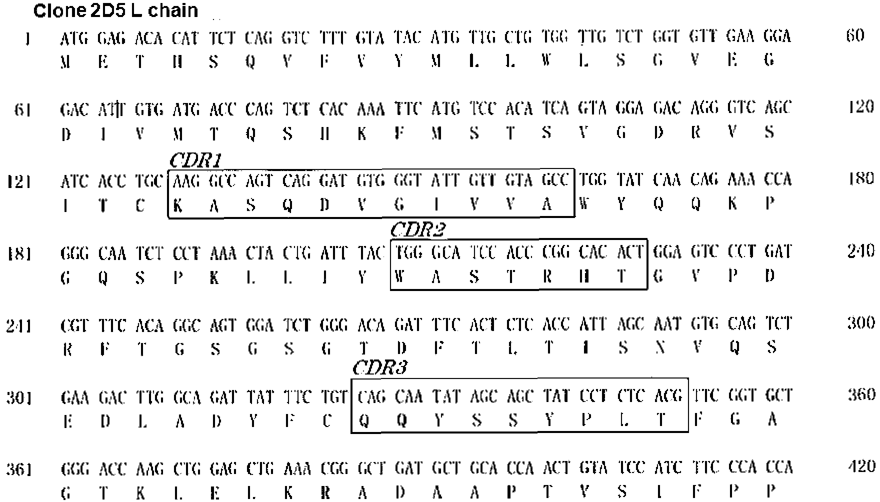

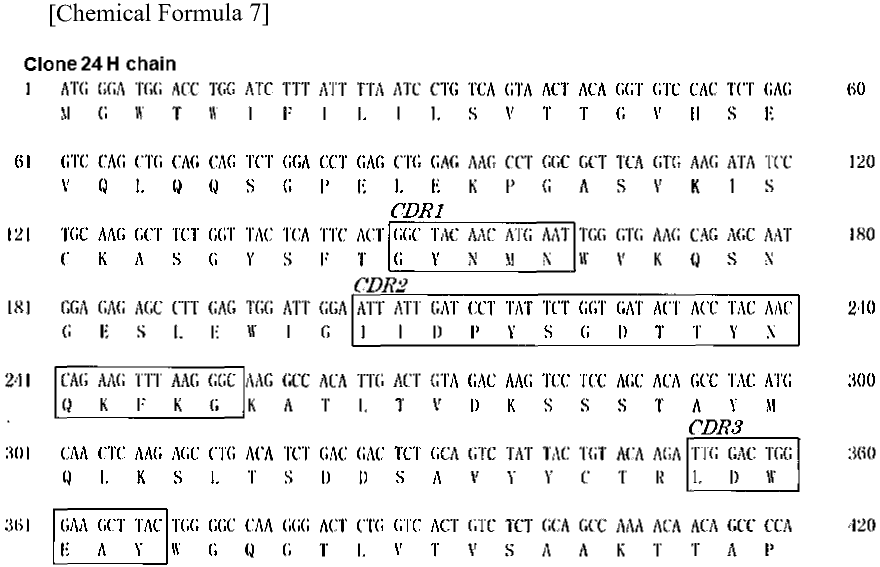

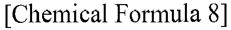

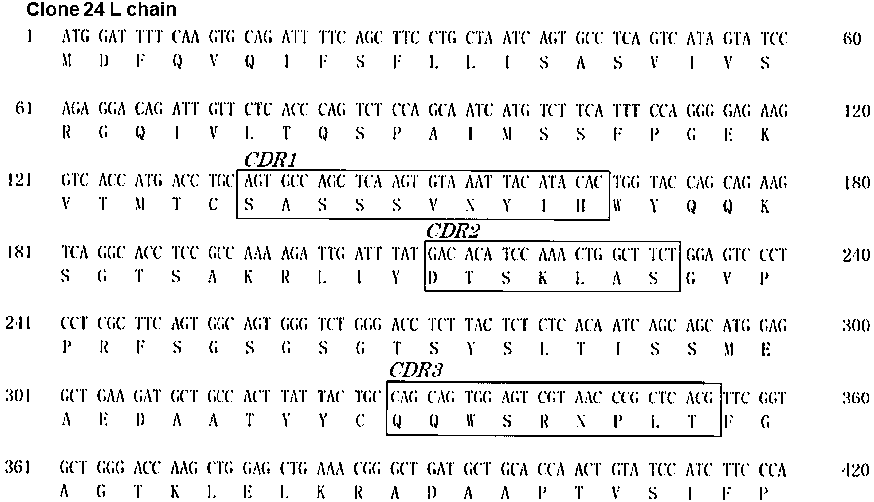

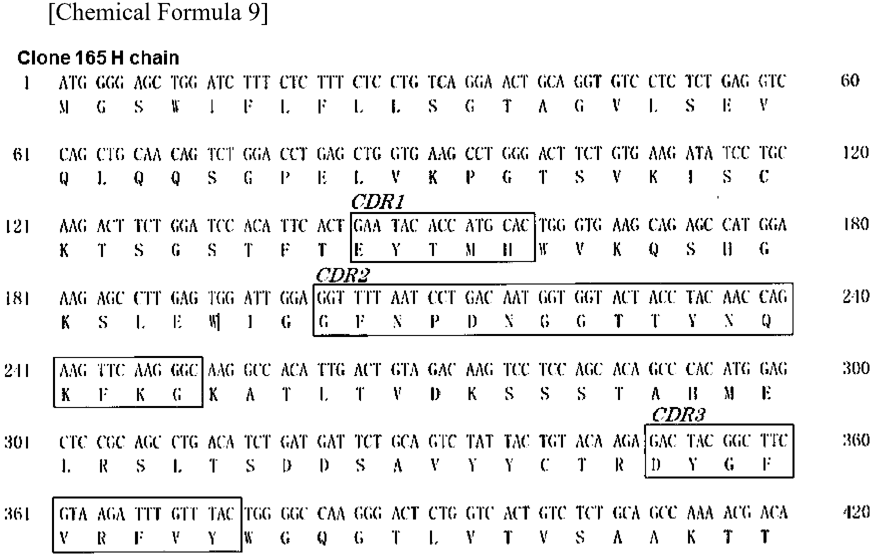

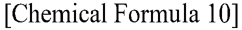

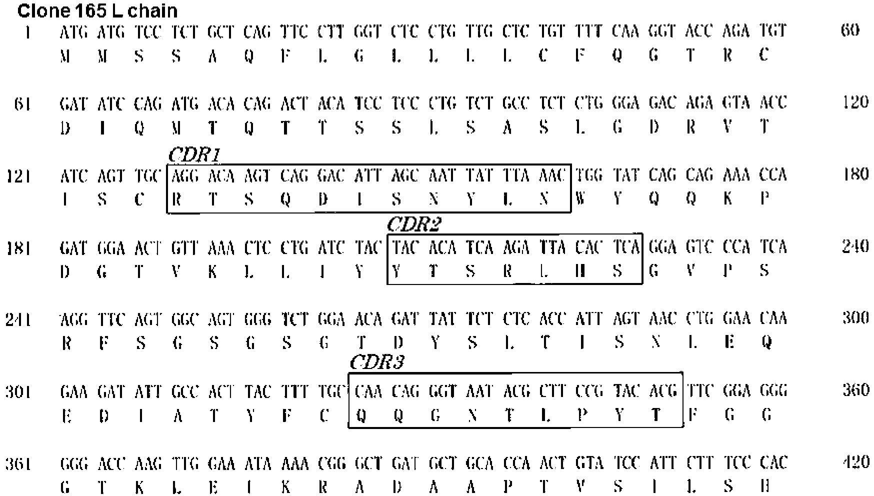

### Chimerization of Antibodies

The antibody gene fragments of clones 577, 2D5, 24, and 165 were each amplified by PCR and cloned into pcDNA3.3 vectors (Thermo Fisher) into which human antibody constant regions had been incorporated, to prepare expression vectors. The sequences were confirmed by a DNA sequencer. The prepared expression vectors were introduced into CHO cells using the Expi CHO Expression System (Thermo Fisher), and transient expression of antibodies was performed. At this time, for clones 577, 2D5, 24, and 165, two types of antibody formats with IgG1 and IgG4 constant regions and Fc regions were prepared, and monoclonal antibody production of each clone in IgG1 and IgG4 formats was performed in CHO cells. More specifically, the heavy chain variable region of each clone was linked with a human IgG1 heavy chain constant region and Fc region having the amino acid sequence set forth in SEQ ID NO: 4, and the light chain variable region of each clone was linked with a light chain constant region having the amino acid sequence set forth in SEQ ID NO: 6, to obtain chimeric antibodies in the IgG1 format. In addition, the heavy chain variable region of each clone was linked with a human IgG4 heavy chain constant region and Fc region having the amino acid sequence set forth in SEQ ID NO: 5, and the light chain variable region of each clone was linked with a light chain constant region having the amino acid sequence set forth in SEQ ID NO: 6, to obtain chimeric antibodies in the IgG4 format.

The obtained antibodies were recovered from culture supernatants and subjected to affinity purification with Protein A and purification by gel filtration column chromatography for use in experiments.

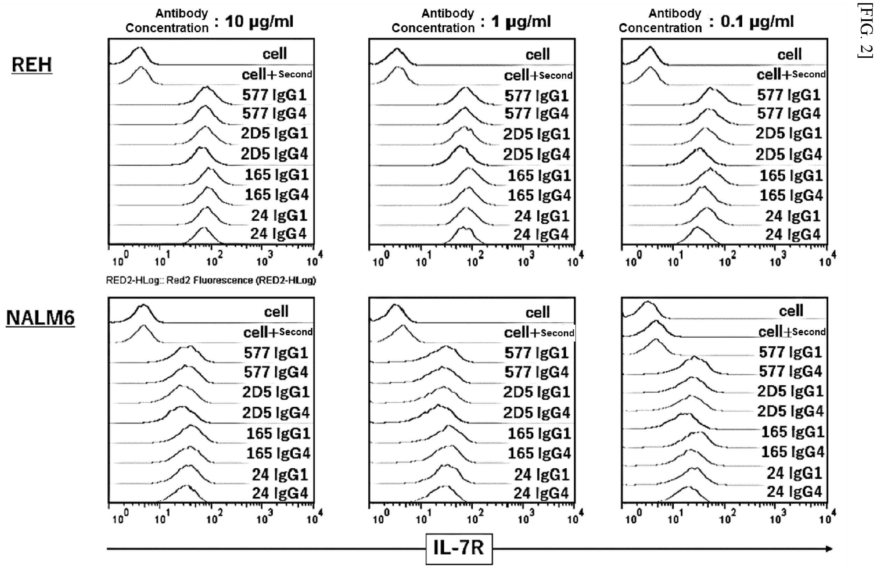

The binding of anti-human IL-7R antibody clones 577, 2D5, 24, and 165 in IgG1 and IgG4 formats to human IL-7R-positive pre-B cell line NALM6 and IL-7R strongly-positive cell line REH was measured by Flow Cytometry (see FIG. 2).

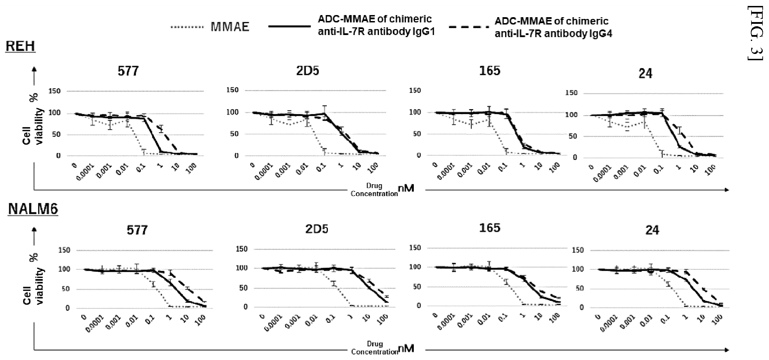

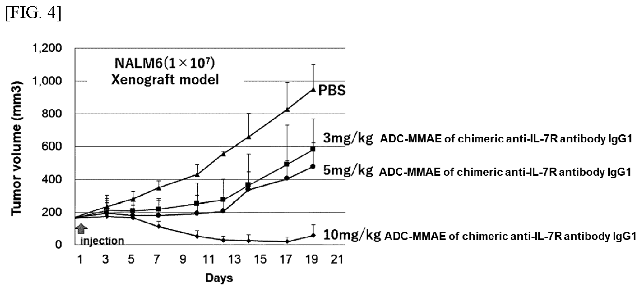

Furthermore, Antibody-drug conjugates (ADCs) with MMAE as a payload were prepared for each, and in vitro cell-killing effects against NALM6 and REH were confirmed. Strong cell-killing effects were observed in all cases (see FIG. 3). Furthermore, for the clone 577 chimeric antibody in the IgG1 format, a strong antitumor effect was observed in an animal model of NALM6 (see FIG. 4). When ADCs with PNU-159682 as a payload were prepared and in vitro cell-killing effects were similarly confirmed, markedly higher cell-killing effects were observed compared to ADCs with MMAE as a payload.

PNU-159682 has the following structure, and the prepared ADCs have the following structures. The drug-to-antibody ratio of the prepared ADCs was 3 to 4.

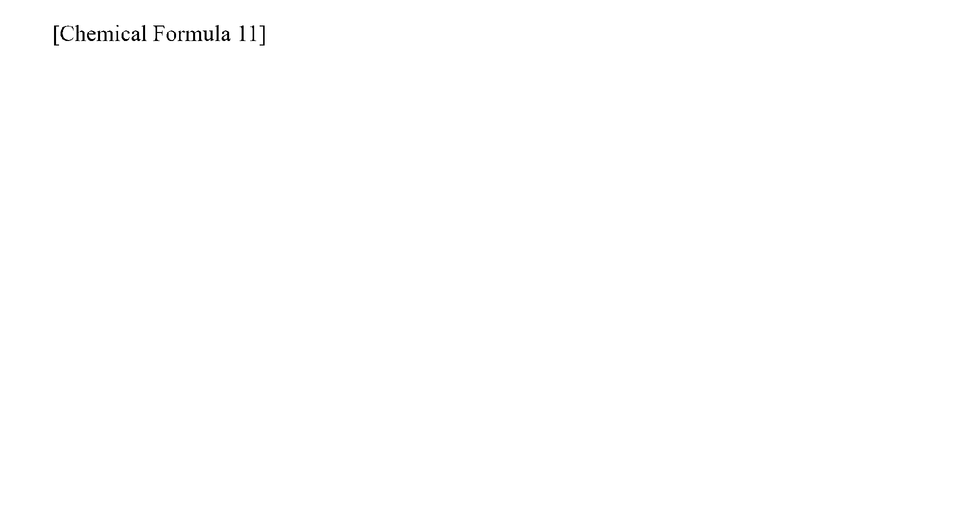

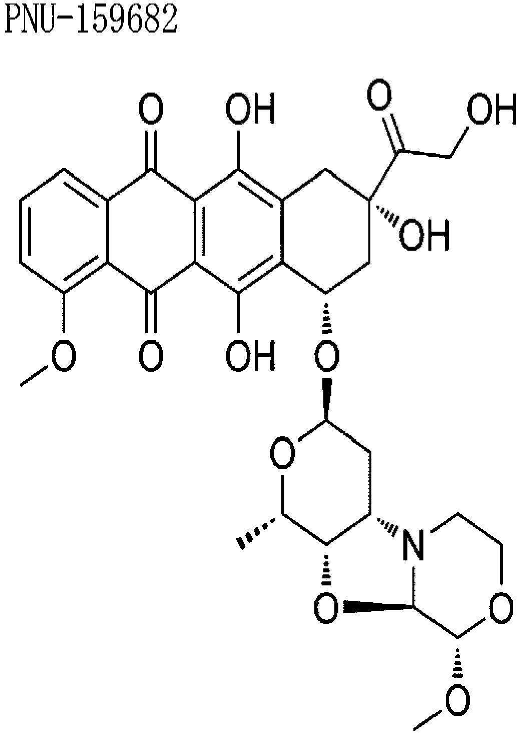

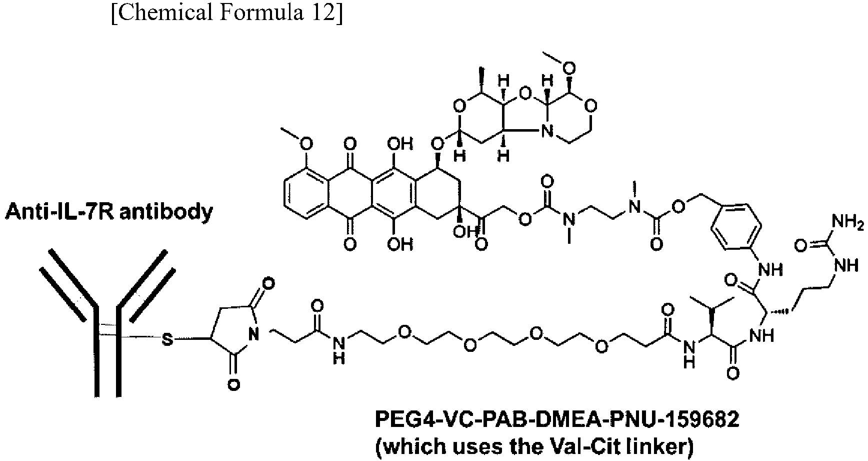

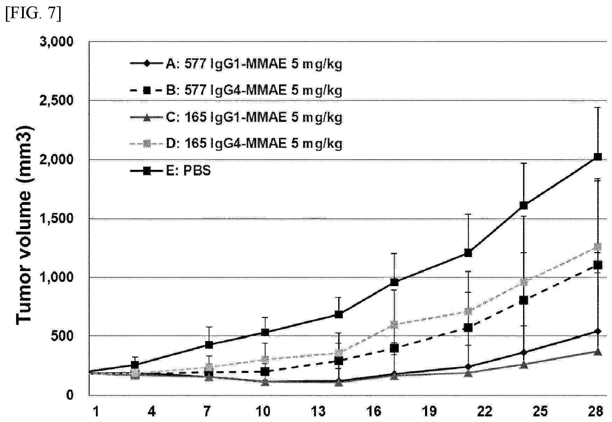

In addition to the clone 577 chimeric antibody, for the clone 165 chimeric antibody, IgG1 and IgG4 formats were prepared for each, and ADCs with MMAE as a payload were prepared. When the antitumor effect was confirmed in an animal model of NALM6, both clone 577 and clone 165 showed stronger antitumor effects in the IgG1 format than in the IgG4 format (FIG. 7).

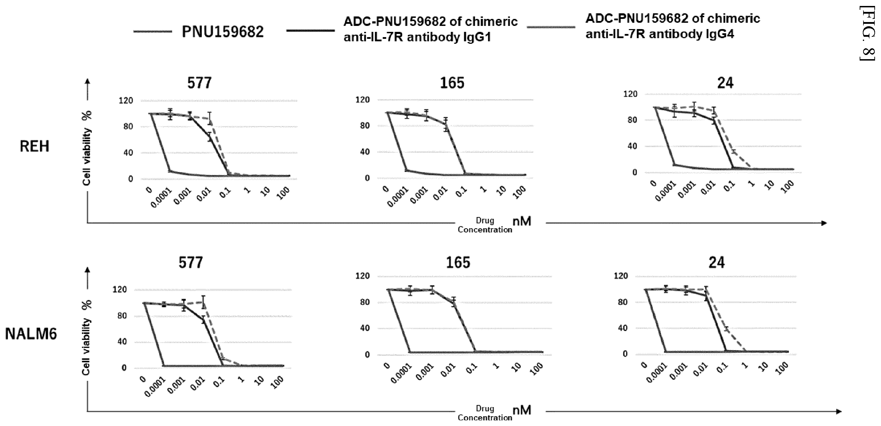

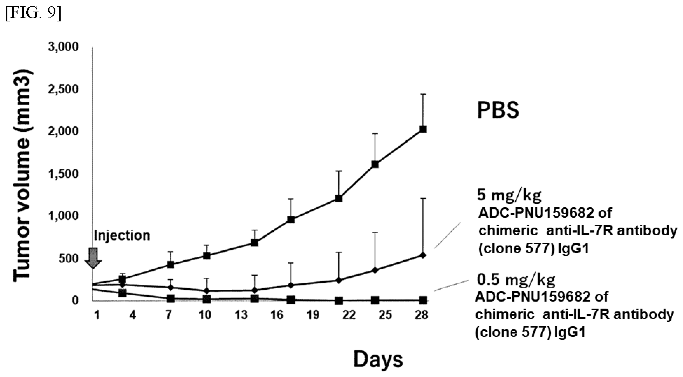

Furthermore, for the clone 577 chimeric antibody, the clone 165 chimeric antibody, and the clone 24 chimeric antibody, ADCs with PNU-159682 as a payload were prepared, and in vitro cell-killing effects against NALM6 and REH were confirmed. Strong in vitro cell-killing effects were observed in all cases (FIG. 8). Note that it is normal for free cytotoxic agents to act at lower concentrations than ADCs, and this does not mean that the effect of ADCs is low. Furthermore, when the antitumor effect of two types of ADCs, an ADC with MMAE as a payload and an ADC with PNU-159682 as a payload, was confirmed for the clone 577 chimeric antibody in the IgG1 format in an animal model of NALM6, the latter showed a stronger antitumor effect at a lower dose (0.5 mg/kg) than the former (FIG. 9).

### Humanization of Antibodies

Humanization modifications were performed on the chimerized clones 577 and 165. Amino acid variants were designed in multiple types for each clone according to the literature (Methods Mol Biol. 2014;1060:123-37. doi: 10.1007/978-1-62703-586-6), and gene fragments were prepared by artificial genes. Cloning and purification were performed by the same methods as for chimerization, and the products were used in experiments. In addition, as a comparison, a previously reported anti-IL-7R antibody clone B12 (Leukemia. 2019; 33(9): 2155-2168.) chimerized into the IgG1 format was prepared and used.

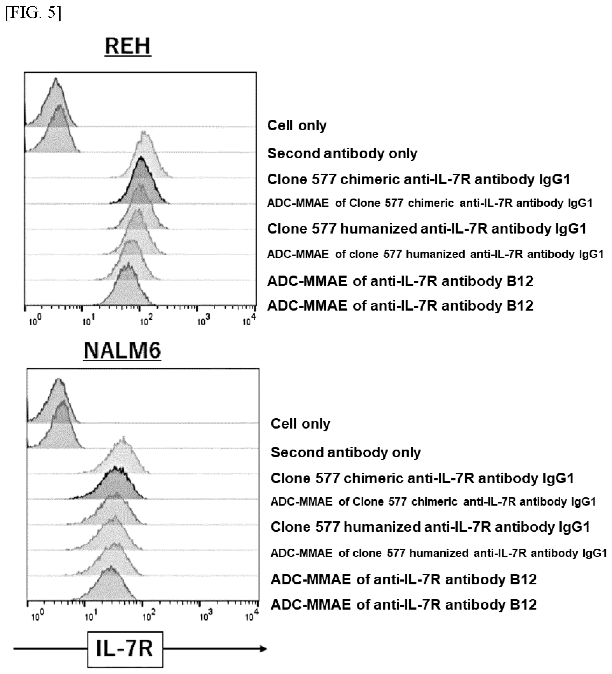

The binding of the anti-human IL-7R antibody clone 577 in the IgG1 format to IL-7R-positive cell line NALM6 and IL-7R strongly-positive cell line REH was measured by Flow Cytometry (see FIG. 5).

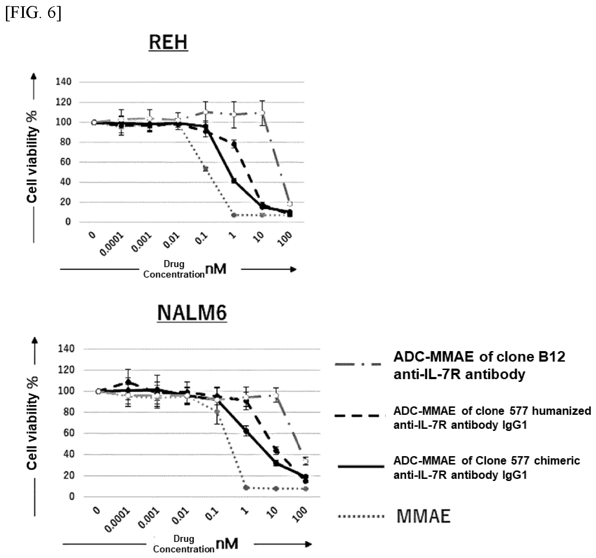

Furthermore, Antibody-drug conjugates (ADCs) with MMAE as a payload were prepared. Specifically, the above anti-human IL-7R antibody clones were treated in cysteine at 37 degrees C for 30 minutes for reduction, and VcMMAE (MedChemExpress, USA) was reacted with the reduced antibody clones at 4 degrees C overnight to obtain ADCs. Higher in vitro cell-killing effects than the previously reported clone B12 (Leukemia. 2019 doi: 10.1038/s41375-019-0434-8) were confirmed against NALM6 and REH. Strong cell-killing effects were observed in all cases
(see FIG. 6).

All documents cited herein are incorporated herein by reference in their entirety.

**[Table 2]**

| **Table 2 Contents of Sequence Listing** | | |
|---|---|---|
| **SEQ ID NO** | **Name** | **Sequence** |
| **1** | **Forward primer** | **CTAATACGACTCACTATAGGGCAAGCAGTGGTATCAACGCAGAGT** |
| **2** | **Reverse primer 1** | **GGACTCTGGGATCATTTACCCGGAGAGT** |
| **3** | **Reverse primer 2** | **CCTTAGGAGGGAAGATIGGAAGGAGCT** |
| **4** | **Human IgG1 CH+Fe** | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKKVEPKSCDKTHTCPPCPAPELLGGPSVFLFPPKPKDTLMISRTPE VTCVVVDVSHEDPEVKFNWYVDGVEVHNAKTKPREEQYNSTYRVVS VLTVLHQDWLNGKEYKCKVSNKALPAPIEKTISKAKGQPREPQVYTL ASTKGPSVFPLAPCSRSTSESTAALGCLVKDYFPEPVTVSWNSGALT |
| **5** | **Human IgG4 CH+Fc** | SGVHTFPAVLQSSGLYSLSSVVTVPSSSLGTKTYTCNVDHKPSNTKV DKRVESKYGPPCPPCPAPEFLGGPSVFLFPPKPKDTLMISRTPEVTC VVVDVSQEDPEVQFNWYVDGVEVHNAKTKPREEQFNSTYRVVSVLT VLHQDWLNGKEYKCKVSNKGLPSSIEKTISKAKGQPREPQVYTLPPS |
| **6** | **Human IgG CL** | LRRTVAAPSVFIFPPSDEQLKSGTASVVCLLNNFYPREAKVQWKVDN ALQSGNSQESVTEQDSKDSTYSLSSTLTLSKADYEKHKVYACEVTHQ |
| **7** | **Human IgG1 H-FR1** | **EVQLVESGGGLVQPGGSLRLSCAASGFNIK** |
| **8** | **Human IgG1 H-FR2** | **WVRQAPGKGLEWVA** |
| **9** | **Human IgG1 H-FR3** | **RFTISADTSKNTAYLQMNSLRAEDTAVYYCSR** |
| **10** | **Human IgG1 H-FR4** | **WGQGTLVTVSS** |
| **11** | **Human IgG1 L-FR1** | **DIQMTQSPSSLSASVGDRVTITC** |
| **12** | **Human IgG1 L-FR2** | **WYQQKPGKAPKLLIY** |
| **13** | **Human IgG1 L-FR3** | **GVPSRFSGSRSGTDFTLTISSLQPEDFATYYC** |
| **14** | **Human IgG1 L-FR4** | **FGQGTKVE** |
| **15** | **577 VH chain** | EVQLQQSGAELVKPGASVKLSCTVSGFNIKDTYIHWVKQRPEQGLEW IGRIDPASDDTKYDPKFQGKATLTADTSSNTAYLQLSSLTSEDTAVYY |
| **16** | **577 VL chain** | ETTVTQSPASLSMAMGEKVTIRCMTSTDINDDMNWYQQKPGEPPKL LISEGNTLRPGVPSRFSSSGYSTDFFFTIEYMLSEDVADYFCLQTDNL |
| **17** | **577 HCDR1** | **DTYIH** |
| **18** | **577 HCDR2** | **RIDPASDDTKYDPKFQG** |
| **19** | **577 HCDR3** | **RVYDYLDY** |
| **20** | **577 LCDR1** | **MTSTDINDDMN** |
| **21** | **577 LCDR2** | **EGNTLRP** |
| **22** | **577 LCDR3** | **LQTDNLPLT** |
| **23** | **2D5 VH chain** | QVQLQQSGAELVKPGASVKLSCKASGYTFTNYYMYWVKQRPGQGL EWIGEINPSNGGTNFNEKFKSKATLTVDKSSSTAYMQLSSLTSEDSAV |
| **24** | **2D5 VL chain** | DIVMTQSHKFMSTSVGDRVSITCKASQDVGIVVAWYQQKPGQSPKLL IYWASTRHTGVPDRFTGSGSGTDFTLTISNVQSEDLADYFCQQYSSY |

**[Table 2-2]**

| | | |
|---|---|---|
| **25** | **2D5 HCDR1** | **NYYMY** |
| **26** | **2D5 HCDR2** | **EINPSNGGTNFNEKFKS** |
| **27** | **2D5 HCDR3** | **RSFAY** |
| **28** | **2D5 LCDR1** | **KASQDVGIVVA** |
| **29** | **2D5 LCDR2** | **WASTRHT** |
| **30** | **2D5 LCDR3** | **QQYSSYPLT** |
| **31** | **24 VH chain** | EVQLQQSGPELEKPGASVKISCKASGYSFTGYNMNWVKQSNGESLE WIGIIDPYSGDTTYNQKFKGKATLTVDKSSSTAYMQLKSLTSDDSAVY |
| **32** | **24 VL chain** | QIVLTQSPAIMSSFPGEKVTMTCSASSSVNYIHWYQQKSGTSPKRLIY DTSKLASGVPPRFSGSGSGTSYSLTISSMEAEDAATYYCQQWSRNPL |
| **33** | **24 HCDR1** | **GYNMN** |
| **34** | **24 HCDR2** | **IIDPYSGDTTYNQKFKG** |
| **35** | **24 HCDR3** | **LDWEAY** |
| **36** | **24 LCDR1** | **SASSSVNYIH** |
| **37** | **24 LCDR2** | **DTSKLAS** |
| **38** | **24 LCDR3** | **QQWSRNPLT** |
| **39** | **165 VH chain** | EVQLQQSGPELVKPGTSVKISCKTSGSTFTEYTMHWVKQSHGKSLE WIGGFNPDNGGTTYNQKFKGKATLTVDKSSSTAHMELRSLTSDDSA |
| **40** | **165 VL Chain** | DIQMTQTTSSLSASLGDRVTISCRTSQDISNYLNWYQQKPDGTVKLLI YYTSRLHSGVPSRFSGSGSGTDYSLTISNLEQEDIATYFCQQGNTLPY |
| **41** | **165 HCDR1** | **EYTMH** |
| **42** | **165 HCDR2** | **GFNPONGGTTYNQKFKG** |
| **43** | **165 HCDR3** | **DYGFVRFVY** |
| **44** | **165 LCDR1** | **RTSQDISNYLN** |
| **45** | **165 LCDR2** | **YTSRLHS** |
| **46** | **165 LCDR3** | **QQGNTLPYT** |
| **47** | **Humanized 577 H chain** | EVQLVQSGAEVKKPGASVKVSCTVSGYTIKDTYIHWVKQAPEQGLEW MGRIDPASDDTKYDPKFQGKVTLTADTSINTAYLQLSSLTSEDTAVY |
| **48** | **Humanized 577 L chain** | ETTLTQSPAFMSATMGDKVTISCMTSTDINDDMNWYQQKPGEPPILI IQEGNTLRPGIPPRFSGSGYGTDFTFTINNMLSEDAADYFCLQTDNLP |
| **49** | **Humanized 165 H chain** | EVQLVQSGAEVKKPGASVKVSCKVSGYTFTEYTMHWVKQAPGKGLE WMGGFNPDNGGTTYNQKFKGKVTLTVDTSTSTAYMELRSLRSEDT |
| **50** | **Humanized 165 L chain** | DIQMTQSPSSLSASLGDRVTITCRTSQDISNYLNWYQQKPGEAVILIIQ YTSRLHSGVPSRFSGSGSGTDYTFTISNLQQEDIATYYCQQGNTLPYT |
| **51** | **Human IgG1 H-FR1 (1)** | **EVQLVQSGAEVKKPGASVKVSCTVSGYTIK** |
| **52** | **Human IgG1 H-FR2 (1)** | **WVKQAPEQGLEWMG** |
| **53** | **Human IgG1 H-FR3 (1)** | **KVTLTADTSINTAYLQLSSLTSEDTAVYYCVR** |
| **54** | **Human IgG1 H-FR4 (1)** | **WGQGTTVTVSS** |
| **55** | **Human IgG1 L-FR1 (1)** | **ETTLTQSPAFMSATMGDKVTISC** |
| **56** | **Human IgG1 L-FR2 (1)** | **WYQQKPGEPPILIIQ** |
| **57** | **Human IgG1 L-FR3 (1)** | **GIPPRFSGSGYGTDFTFTINNMLSEDAADYFC** |
| **58** | **Human IgG1 L-FR4 (1)** | **FGGGTKLE** |
| **59** | **Human IgG1 H-FR1 (2)** | **EVQLVQSGAEVKKPGASVKVSCKVSGYTFT** |
| **60** | **Human IgG1 H-FR2 (2)** | **WVKQAPGKGLEWMG** |
| **61** | **Human IgG1 H-FR3 (2)** | **KVTLTVDTSTSTAYMELRSLRSEDTAVYYCAR** |
| **62** | **Human IgG1 H-FR4 (2)** | **WGQGTLVTVSA** |
| **63** | **Human IgG1 L-FR1 (2)** | **DIQMTQSPSSLSASLGDRVTITC** |
| **64** | **Human IgG1 L-FR2 (2)** | **WYQQKPGEAVILIIQ** |
| **65** | **Human IgG1 L-FR3 (2)** | **GYPSRFSGSGSGTDYTFTISNLQQEDIATYYC** |
| **66** | **Human IgG1 L-FR4 (2)** | **FGGGTKVE** |

## Claims

1. An isolated monoclonal antibody or an antigen-binding fragment thereof that binds to human interleukin-7 receptor (IL-7R), wherein the antibody and the antigen-binding fragment thereof comprise:
(1) a heavy chain variable region comprising heavy chain CDR1 having the amino acid sequence set forth in SEQ ID NO: 17, heavy chain CDR2 having the amino acid sequence set forth in SEQ ID NO: 18, and heavy chain CDR3 having the amino acid sequence set forth in SEQ ID NO: 19, and a light chain variable region comprising light chain CDR1 having the amino acid sequence set forth in SEQ ID NO: 20, light chain CDR2 having the amino acid sequence set forth in SEQ ID NO: 21, and light chain CDR3 having the amino acid sequence set forth in SEQ ID NO: 22;
(2) a heavy chain variable region comprising heavy chain CDR1 having the amino acid sequence set forth in SEQ ID NO: 25, heavy chain CDR2 having the amino acid sequence set forth in SEQ ID NO: 26, and heavy chain CDR3 having the amino acid sequence set forth in SEQ ID NO: 27, and a light chain variable region comprising light chain CDR1 having the amino acid sequence set forth in SEQ ID NO: 28, light chain CDR2 having the amino acid sequence set forth in SEQ ID NO: 29, and light chain CDR3 having the amino acid sequence set forth in SEQ ID NO: 30;
(3) a heavy chain variable region comprising heavy chain CDR1 having the amino acid sequence set forth in SEQ ID NO: 33, heavy chain CDR2 having the amino acid sequence set forth in SEQ ID NO: 34, and heavy chain CDR3 having the amino acid sequence set forth in SEQ ID NO: 35, and a light chain variable region comprising light chain CDR1 having the amino acid sequence set forth in SEQ ID NO: 36, light chain CDR2 having the amino acid sequence set forth in SEQ ID NO: 37, and light chain CDR3 having the amino acid sequence set forth in SEQ ID NO: 38; or
(4) a heavy chain variable region comprising heavy chain CDR1 having the amino acid sequence set forth in SEQ ID NO: 41, heavy chain CDR2 having the amino acid sequence set forth in SEQ ID NO: 42, and heavy chain CDR3 having the amino acid sequence set forth in SEQ ID NO: 43, and a light chain variable region comprising light chain CDR1 having the amino acid sequence set forth in SEQ ID NO: 44, light chain CDR2 having the amino acid sequence set forth in SEQ ID NO: 45, and light chain CDR3 having the amino acid sequence set forth in SEQ ID NO: 46.

2. The antibody or the antigen-binding fragment thereof according to Claim 1, wherein the antibody or the antigen-binding fragment thereof is a human chimeric antibody or an antigen-binding fragment thereof.

3. The antibody or the antigen-binding fragment thereof according to Claim 1, wherein the antibody or the antigen-binding fragment thereof is a humanized antibody or an antigen-binding fragment thereof.

4. The antibody or the antigen-binding fragment thereof according to Claim 3, wherein the humanized antibody has a heavy chain constant region, an Fc region, and a light chain constant region of a subclass selected from the group consisting of human IgG1, IgG2, and IgG4.

5. An antibody-drug conjugate comprising an antibody or an antigen-binding fragment thereof and a drug, wherein the antibody or fragment is linked to the drug via a linker, and the antibody or fragment comprises the antibody or the antigen-binding fragment thereof according to any one of Claims 1 to 4.

6. A pharmaceutical composition comprising the antibody or the antigen-binding fragment thereof according to any one of Claims 1 to 4.

7. The pharmaceutical composition according to Claim 6, wherein the antibody or the antigen-binding fragment thereof has antibody-dependent cellular cytotoxicity activity and/or complement-dependent cytotoxicity activity.

8. A pharmaceutical composition comprising the antibody-drug conjugate according to Claim 5.

9. The pharmaceutical composition according to any one of Claims 6 to 8, for use in treating cancer in a subject in need thereof.

10. The pharmaceutical composition according to any one of Claims 6 to 8, for use in treating a disease in a subject in need thereof, wherein the disease is selected from the group consisting of cancer, immune diseases, inflammatory diseases, and diseases resulting from cancer, immune diseases, or inflammatory diseases.
